(19) Europäisches Patentamt
European Patent Office
Office européen des brevets

(11) Publication number: **0 071 434**
**B1**

(12) # EUROPEAN PATENT SPECIFICATION

(45) Date of publication of patent specification: **28.01.87**

(21) Application number: **82303911.0**

(22) Date of filing: **23.07.82**

(51) Int. Cl.[4]: **C 07 C 129/14,**
C 07 D 401/06,
C 07 D 249/14,
C 07 D 239/46,
C 07 D 471/04,
C 07 D 257/06, C 07 C 93/14,
C 07 C 93/187

(54) **Heterocyclic derivatives.**

(30) Priority: **24.07.81 GB 8122875**
**16.02.82 GB 8204483**
**16.02.82 GB 8204484**
**25.02.82 GB 8205596**

(43) Date of publication of application:
**09.02.83 Bulletin 83/06**

(45) Publication of the grant of the patent:
**28.01.87 Bulletin 87/05**

(84) Designated Contracting States:
**BE CH DE FR GB IT LI NL**

(56) References cited:
**EP-A-0 013 071**
**EP-A-0 029 306**
**DE-A-2 821 410**

**The file contains technical information submitted after the application was filed and not included in this specification**

(73) Proprietor: **GLAXO GROUP LIMITED**
**Clarges House 6-12 Clarges Street**
**London W1Y 8DH (GB)**

(72) Inventor: **Hayes, Roger**
**5, Sandringham Road**
**Potters Bar Hertfordshire (GB)**
Inventor: **Bays, David Edmund**
**9, Windmill Field**
**Ware Hertfordshire (GB)**
Inventor: **Mackinnon, John Wilson Macfarlane**
**31 Trapstyle Road**
**Ware Hertfordshire (GB)**
Inventor: **Carey, Linda**
**3 The Lawn Close**
**Melbourne Royston Hertfordshire (GB)**
Inventor: **Blatcher, Philip**
**43, Trafalgar Avenue**
**Broxbourne Hertfordshire (GB)**

(74) Representative: **Marchant, James Ian et al**
**Elkington and Fife High Holborn House**
**52/54 High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 071 434 B1

**Description**

This invention relates to novel heterocyclic derivatives having action on histamine receptors, to processes for preparation thereof, to pharmaceutical compositions containing them and to their use in therapeutics.

Certain novel heterocyclic derivatives have now been found which have potent activity as $H_2$-antagonists. These compounds, which are more particularly described below, for example show inhibition of the secretion of gastric acid when this is stimulated via histamine receptors (Ash and Schild, Brit. J. Pharmacol. Chemother, 1966, *27*, 427). Their ability to do so can be demonstrated in the perfused rat stomach using the method described in British Patent Specification Number 1565966, modified by the use of sodium pentobarbitone (50 mg/kg) as anaesthetic instead of urethane, and in conscious dogs equipped with Heidenhain pouches using the method described by Black *et al*, Nature 1972 *236*, 385. Furthermore, the compounds antagonise the effect of histamine on the concentration frequency of isolated guinea pig right atrium.

Prior art compounds with histamine $H_2$-blocking activity are known from EP—A—0 082 376 (Merck priority 21.12.81 US 332 691), EP—A—0 029 306, EP—A—0 016 565 and EP—A—0 049 049 (Glaxo), EP—A—0013 071 (Smith Kline) and DE—A—2 821 410 (Allen & Hanburys).

Compounds with histamine $H_2$-blocking activity may be used in the treatment of conditions where there is an advantage in lowering gastric acidity, particularly in gastric and peptic ulceration, as a prophylatic measure in surgical procedures, and in the treatment of allergic and inflammatory conditions where histamine is a known mediator. Thus they may be used for example, either alone or in a combination with other active ingredients in the treatment of allergic and inflammatory conditions of the skin.

The present invention provides compounds of the general formula (I).

$$R_1R_2NAlkQO(CH_2)_n\underset{}{\overset{OR_5}{\overset{|}{C}}}H(CH_2)_mNH{-}Z \qquad (I)$$

and physiologically acceptable salts and hydrates thereof in which

$R_1$ represents hydrogen, $C_{1-14}$ alkyl, $C_{3-8}$ cycloalkyl, $C_{2-6}$ alkenyl, $C_{2-6}$ alkynyl, aryl $C_{1-6}$ alkyl, trifluoro $C_{1-6}$ alkyl, heteroaralkyl or $C_{1-6}$ alkyl substituted by $C_{3-8}$ cycloalkyl, hydroxy, $C_{1-6}$ alkoxy, amino, $C_{1-6}$ alkylamino or di $C_{1-6}$ alkylamino; and

$R_2$ represents hydrogen or $C_{1-4}$ alkyl group; or

$R_1$ and $R_2$ together with the nitrogen atom to which they are attached form a 5 to 10 membered ring which may be saturated or may contain at least one double bond, may be unsubstituted or may be substituted by one or more $C_{1-3}$ alkyl groups or a hydroxy group and/or may contain another heteroatom selected from oxygen or sulphur;

Alk represnts a straight or branched alkylene chain of 1 to 3 carbon atoms;

Q represents a benzene ring in which incorporation into the rest of the molecule is through bonds at the 1- and 3- or 1- and 4-positions;

$R_5$ represents hydrogen or acyl;

n and m, which may be the same or different, are each 1 or 2;

Z represents one of the groups;

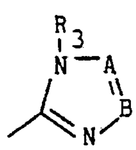 or 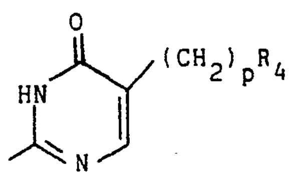 or 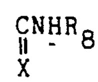

where X represents NCN, $NSO_2$Methyl, $NSO_2$Phenyl, or $CHNO_2$;

$R_8$ represents $C_{1-6}$ alkyl;

A represents N and B represents $CR_7$; or

A represents $CR_7$ and B represents N; or

A and B both represent N;

$R_3$ represents hydrogen, $C_{1-6}$ alkyl, $C_{3-6}$ alkenyl, aryl $C_{1-6}$ alkyl, hydroxy $C_{2-6}$ alkyl, $C_{1-6}$ alkoxy $C_{2-6}$ alkyl or $C_{1-4}$ alkanoyloxy $C_{2-6}$ alkyl;

$R_7$ represents hydrogen, $C_{1-6}$ alkyl, $C_{3-6}$ alkenyl, aryl $C_{1-6}$ alkyl, acyloxy $C_{1-6}$ alkyl, $C_{1-6}$ alkylthio $C_{1-6}$ alkyl, arylthio $C_{1-6}$ alkyl, aryloxy $C_{1-6}$ alkyl, aryl $C_{1-6}$ alkyloxy $C_{1-6}$ alkyl, or the group $(CH_2)_qR_6$ where q is zero, 1, 2, 3, 4, 5 or 6 and the alkylene chain $(CH_2)_q$ may be straight or branched, and

$R_6$ is hydroxy, $C_{1-6}$ alkoxy, nitro, heteroaryl, tetrahydropyranyloxy, or $CH_2NHC(=X)NHR_9$ where X is as defined above and $R_9$ is $C_{1-6}$ alkyl;

or $R_6$ is the group $NR_{10}R_{11}$, where $R_{10}$ is hydrogen or $C_{1-6}$ alkyl; and $R_{11}$ is hydrogen, $C_{1-6}$ alkyl, $C_{3-6}$ alkenyl, aryl, aryl $C_{1-6}$ alkyl, or heteroaralkyl, or $R_{11}$ is the group $SO_2R_{12}$ where $R_{12}$ is $C_{1-6}$ alkyl; or $R_{11}$ is the

group $COR_{12}$ where $R_{13}$ is hydrogen, $C_{1-6}$ alkyl, aryl, aryl $C_{1-6}$ alkyl, $C_{1-6}$ alkoxy, halomethyl, heteroaryl, heteroaralkyl or the group $NHR_{14}$ where $R_{14}$ is hydrogen, $C_{1-6}$ alkyl, $C_{3-8}$ cycloalkyl, aryl or aryl $C_{1-6}$ alkyl; or $R_{10}$ and $R_{11}$ together represent the group $=CR_{15}R_{16}$ where $R_{15}$ represents aryl or heteroaryl and $R_{16}$ represents hydrogen or $C_{1-6}$ alkyl;

or $R_6$ is the group $SO_2R_{17}$ in which $R_{17}$ is hydroxy, $C_{1-6}$ alkyl, aryl or the group $NR_{18}R_{19}$ where $R_{18}$ and $R_{19}$, which may be the same or different, each represent hydrogen or $C_{1-6}$ alkyl;

or $R_6$ is the group $COR_{20}$ where $R_{20}$ is hydrogen, hydroxy, $C_{1-6}$ alkoxy, aryloxy, aryl $C_{1-6}$ alkyloxy, $C_{1-6}$ alkyl, aryl, aryl $C_{1-6}$ alkyl, or the group $NR_{21}R_{22}$ where $R_2$ is hydrogen or $C_{1-6}$ alky optionally substituted by a hydrogen or $C_{1-6}$ alkoxy group; and $R_{22}$ is hydrogen, $C_{1-6}$ alkyl (optionally substituted by a hydroxy or $C_{1-6}$ alkoxy group), $C_{3-6}$ alkenyl, aryl, aryl $C_{1-6}$ alkyl or $C_{3-8}$ cycloalkyl, or $NR_{21}R_{22}$ forms a 5 to 8 membered ring which may contain another heteroatom, e.g. oxygen, or a double bond and/or may be substituted by hydroxy or one or two $C_{1-3}$ alkyl groups; or $R_6$ is the group $CR_{23}=RNR_{28}$ where $R_{23}$ is hydrogen, $C_{1-6}$ alkyl, aryl or aryl $C_{1-6}$ alkyl and $R_{24}$ is hydroxy, $C_{1-6}$ alkoxy, aryl $C_{1-6}$ alkyloxy or $-NHC(=Y)NH_2$ where Y is oxygen or sulphur;

or when A represents $CR_7$ and B represents N, the groups $R_2$ and $R_7$ taken together represent $-(CH=CH)_2-$ or $-(CH_2)_4-$;

with the proviso (1) that when the group $R_6$ contains a carbon atom through which it is linked to the alkylene group $(CH_2)_q$ then the total number of carbon atoms in the resulting chain is not greater than 6 (i.e. q is not greater than 5);

$R_4$ represents phenyl, phenoxy or pyridinyl which may optionally be substituted by one or more halogen, $C_{1-6}$ alkyl, $C_{1-6}$ alkoxy $C_{1-6}$ alkyl or $C_{1-6}$ alkoxy groups or by a methylenedioxy group;

p represents an integer which is 1, 2, or 3; with the following further provisos:

(2) the term aryl within the definitions of $R_1$, $R_3$, $R_6$ and $R_7$ means phenyl or phenyl substituted by one or more $C_{1-3}$ alkyl groups or halogen atoms;

(3) the term heteroaryl as a group or part of a group within the difinitions of $R_1$ and $R_6$ means a 5 or 6 membered monocyclic ring containing from 1 to 3 heteroatoms selected from oxygen, nitrogen and sulphur, which may be unsubstituted or substituted by $C_{1-3}$ alkyl, $C_{1-3}$ alkoxy, hydroxy $C_{1-6}$ alkyl, amino $C_{1-6}$ alkyl, $C_{1-6}$ alkylamino $C_{1-6}$ alkyl, di $C_{1-6}$ alkylamino $C_{1-6}$ alkyl or halogen;

(4) the alkyl portion of a heteroaralkyl group within the definitions of $R_1$ and $R_6$ is a straight or branched $C_{1-4}$ alkyl chain and the heteroaryl ring is linked to the alkyl portion through a carbon atom;

(5) the term acyl as a group or part of a group within the difinitions of $R_5$ and $R_7$ means an aroyl or aryl $C_{2-7}$ alkanoyl group (in which the term aryl is as defined as in proviso 2) or a $C_{1-6}$ alkanoyl group.

The term "alkyl" as a group or part of a group means that the group is straight or branched, and unless otherwise stated, has preferably 1 to 4 carbon atom, e.g. methyl or ethyl. The term "halomethyl" means a mono-, di- or trihalo substituted methyl group, e.g. trifluoromethyl. Within the definition of the term "aryl" as a group or part of a group, fluorine is an example of a halogen substituent on the phenyl group. Examples of acyloxyalkyl groups include acetoxymethyl, formyloxymethyl, benzoyloxymethyl and phenylacetoxymethyl. Examples of heteroaryl groups are thienyl, pyrrolyl, pyridyl, furyl and thiazolyl. Examples of substituted, heteroaryl rings are thienyl or furyl substituted by $C_{1-3}$ alkyl, amino $C_{1-6}$ alkyl, $C_{1-6}$ alkylamino $C_{1-6}$ alkyl, di $C_{1-6}$ alkylamino $C_{1-6}$ alkyl or hydroxy $C_{1-6}$ alkyl, pyrrolyl substituted by $C_{1-3}$ alkyl, pyridyl substituted by $C_{1-3}$ alkyl, $C_{1-3}$ alkoxy, halogen or hydroxy $C_{1-6}$ alkyl or thiazolyl substituted by $C_{1-3}$ alkyl or hydroxy $C_{1-6}$ alkyl.

According to one aspect of the invention provides compounds of formula (I) in which Z is

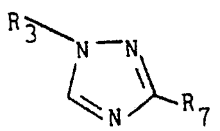

$R_5$ represents hydrogen or a $C_{1-4}$ alkanoyl group;

$R_3$ represents hydrogen, $C_{1-6}$ alkyl, $C_{3-6}$ alkenyl, aryl $C_{1-6}$ alkyl, hydroxy $C_{2-6}$ alkyl, or $C_{1-6}$ alkoxy $C_{2-6}$ alkyl;

$R_7$ is as defined in formula (I) except that $R_6$ does not represent tetrahydropyranyloxy; and

$R_1$, $R_2$, Alk, Q, n and m are as defined in formula (I).

According to a second aspect of the invention provides compounds of formula (I) in which Z is

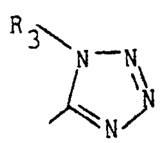

$R_5$ represents hydrogen or a $C_{1-4}$ alkanoyl group; and

$R_1$, $R_2$, $R_3$, Alk, Q, m and n are as defined in formula (I).

According to a third aspect the invention provides compounds of formula (I) in which Z is

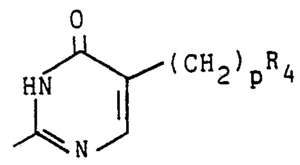

$R_5$ represents hydrogen or a $C_{1-4}$ alkanoyl group; and
$R_1$, $R_2$, $R_4$, Alk, Q, m and n and p are as defined in formula (I).
According to a fourth aspect the invention provides compounds of formula (I) in which Z is

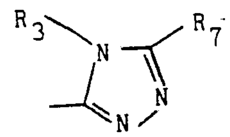

$R_5$ represents hydrogen or a $C_{1-4}$ alkanoyl group;
$R_3$ represents hydrogen, $C_{1-4}$ alkyl, $C_{3-4}$ alkenyl, aryl $C_{1-4}$ alkyl, hydroxy $C_{2-4}$ alkyl or $C_{1-4}$ alkoxy $C_{2-4}$ alkyl;
$R_7$ is as defined in formula (I) except that $R_6$ does not represent tetrahydropyranyloxy; and
$R_1$, $R_2$, Alk, Q, n and m and are as defined in formula (I).
Preferred compounds of formula (I) are those in which
$R_1$ represents $C_{1-8}$ alkyl (e.g. methyl, propyl, butyl or heptyl), $C_{1-4}$ alkyl substituted by a trifluoromethyl group (e.g. 2,2,2-trifluoroethyl), $C_{2-4}$ alkyl substituted by hydroxy or a di $C_{1-3}$ alkyl amino group (e.g. 3-hydroxypyropyl or dimethylaminoethyl), $C_{5-7}$ cycloalkyl (e.g. cyclohexyl), $C_{3-5}$ alkenyl (e.g. allyl), phenyl $C_{1-3}$ alkyl (e.g. benzyl), or a heteroaryl $C_{1-3}$ alkyl group where the heteroaryl ring contains one heteroatom (e.g. 2-furylmethyl);
$R_2$ represents hydrogen or methyl; or
$R_1R_2N$ represents a 5 to 7 membered ring optionally containing a double bond, an oxygen atom or a $C_{1-6}$ alkyl (e.g. methyl) substituent (e.g. piperidino, morpholino, 4-methylpiperidino, pyrrolidino, hexamethylenimino or tetrahydropyridino);
Alk represents methylene;
$R_3$ represents hydrogen, $C_{1-6}$ alkyl (e.g. methyl or ethyl), hydroxy $C_{2-4}$ alkyl (e.g. hydroxyethyl) or, when A is the group $CR_7$, then $R_3$ and $R_7$ taken together represent $-(CH{=}CH)_2-$ or $-(CH_2)_4$;
Q represents a benzene ring incorporated into the rest of the molecule through bonds at the 1- and 3-positions:
$R_5$ represents hydrogen or $C_{1-6}$ alkanoyl (e.g. acetyl);
n and m both represent 1 or one of n and m represent 2;
$R_7$ represents $C_{1-6}$ alkoxy, $C_{1-6}$ alkyl $C_{1-6}$ alkylthio $C_{1-6}$ alkyl, $C_{1-6}$ alkanoyloxy $C_{1-6}$ alkyl, benzyl, or $-CH{=}NOH$; or
$R_7$ represents the group $(CH_2)_qR_6$ where q is zero, 1, 2 or 3, and $R_6$ represents hydroxyl, $CH_2NHSO_2R_{12}$ (where $R_{12}$ is $C_{1-6}$ alkyl), $CH_2NHC(=X)NHCH_3$ (where X=NCN or $CHNO_2$), $SO_2R_{17}$ (where $R_{17}$ is $C_{1-6}$ alkyl), or $R_6$ represents $COR_{20}$ where $R_{20}$ is hydroxyl or $NR_{21}R_{22}$ and $R_{21}$ and $R_{22}$ independently represent hydrogen or $C_{1-3}$ alkyl or $R_{21}$ and $R_{22}$ together with the nitrogen atom to which they are attached represent a pyrrolidino ring, or
$R_6$ represents the group $NR_{10}R_{11}$ where $R_{10}$ represents hydrogen and $R_{11}$ is hydrogen or $COR_{13}$, where $R_{13}$ represents hydrogen, $C_{1-6}$ alkyl, phenyl, benzyl, $C_{1-6}$ alkoxy, Nh phenyl, or $R_{10}$ and $R_{11}$ together represent the group $=CHR_{15}$ where $R_{15}$ is phenyl or pyridyl;
$R_4$ represents pyridinyl (e.g. 3-pyridinyl) optionally substituted by a $C_{1-4}$ alkyl (e.g. methyl) group; or a phenyl group optionally substituted by a $C_{1-6}$ alkoxy (e.g. methoxy) group;
P represents 1;
$R_8$ represents $C_{1-6}$ alkyl (e.g. methyl);
X represents $CHNO_2$.
When Z represents the group

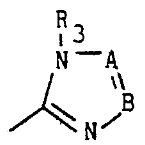

then preferably A is N and B is $CR_7$ or A and B are both N.
A further preferred class of compounds of formula (I) are those of formula (II)

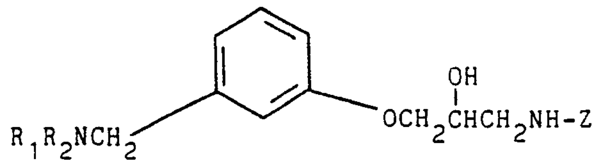

(II)

in which $R_1R_2N$ is dimethylamino, pyrrolidino, piperidino or hexamethylenimino, more preferably piperidino and Z represents

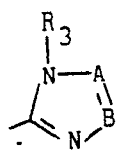 or 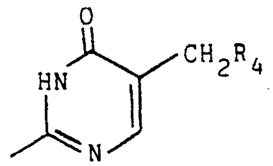 or 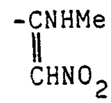

where, when B represents $CR_7$, then $R_3$ is hydrogen or methyl and $R_7$ is hydroxymethyl, amino, $C_{1-6}$ alkanoyloxy, $C_{1-6}$ alkyl (preferably acetoxymethyl), N = CHPh, $(CH_2)_3$ NHC(=$CHNO_2$)NHMe, aminopropyl, methylsulphonylmethyl or acetylamino; or when A is $CR_7$ then $R_3$ is methyl and $R_7$ is amino or $R_3$ and $R_7$ together form the group $\left(CH{=}CH\right)_2$ or $(CH_2)_4$; or when A and B both represent N then $R_3$ is methyl; and $R_4$ is 3-pyridinyl, 6-methyl-3-pyridinyl or 4-methoxyphenyl.

Particularly preferred compounds are

1-(3-amino-1-methyl-1H-1,2,4-triazol-5-yl)amino]-3-[3-(1-piperidinylmethyl)phenoxy)-2-propanol;

5-[[2-hydroxy-3-[3-(1-piperidinylmethyl)phenoxy]propyl]amino)-1-methyl-1*H*-1,2,4-triazole-3-methanol;

1-[(1-methyl-1*H*-tetrazol-5-yl)amino]-3-[3-(1-piperidinylmethyl)-phenoxy]-2-propanol;

2-[[2-hydroxy-3-[3-(1-piperidinylmethyl)phenoxy]propyl]amino]-5-[(6-methyl-3-pyridinyl)methyl]-4-(3*H*)-pyrimidinone;

2-[[2-hydroxy-3-(3-(1-piperidinylmethyl)phenoxy]propyl]amino]-5-(3-pyridinylmethyl)-4-(3H)-pyrimidinone;

2-[[2-hydroxy-3-(3-(1-piperidinylmethyl)phenoxy]propyl]amino]-5-[(4-methoxyphenyl)methyl]-4(3H)-pyrimidinone;

and physiologically acceptable salts thereof.

The invention includes the compounds of formula (I) in the form of physiologically acceptable salts with inorganic and organic acids. Particularly useful salts include hydrochlorides, hydrobromides, sulphates, methanesulphonates, acetates, maleates, succinates, citrate, tartrates, fumarates and benzoates. The compounds of formula (I) and their salts may also form hydrates, which hydrates are also to be considered as part of the invention. The compounds of formula (I) can exhibit tautomerism and the formula is intended to cover all tautomers. Where optical isomers may exist the formula is intended to cover all diastereoisomers and optical enantiomers.

The compounds according to the invention, preferably in the form of a salt may be formulated for administration in any convenient way and the invention includes within its scope pharmaceutical compositions containing at least one compound according to the invention adapted for use in human or veterinary medicine. Such compositions may be formulated in a coventional manner using one or more pharmaceutically acceptable carriers or excipients. Such compositions may also contain if required other active ingredients, e.g. $H_1$-antagonists.

Thus the compounds according to the invention may be formulated for oral, buccal, topical, parenteral or rectal administration. Oral administration is preferred.

For oral administration, the pharmaceutical compositions may take the form of for example, tablets, capsules, powders, solutions, syrups or suspensions prepared by conventional means with acceptable excipients. For buccal administration the compositions may take the form of tablets or lozenges formulated in conventional manner.

The compounds of the invention may be formulated for parenteral administration by bolus injection or continuous infusion. Formulations of injection may be presented in unit dosage form in ampoules, or in multidose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for reconstitution with a suitable vehicle, e.g. sterile pyrogen-free water before use.

The compound of the invention may also be formulated in rectal compositions such as suppositories or retention ememas, e.g. containing conventional suppository bases such as cocoa butter or other glyceride.

For topical application, the compounds of the invention may be formulated as ointments, creams, gels, lotions, powders or sprays in a conventional manner.

For internal administration a convenient daily dosage regime of the compound according to the invention would be 1 to 4 doses to the total of some 5 mg to 1.5 g per day, preferably 5 to 500 mg per day dependent upon the condition of the patient.

It will be appreciated that in the methods of the preparation of compounds of formula (I) given below, for certain reaction steps it may be necessary to protect various reactive substituents in the starting materials for a particular reaction and subsequently to remove the protecting group. Such protection and subsequent deprotection may be particularly pertinent where $R_1$ and/or $R_2$ in intermediates used to prepare compounds of formula (I) are hydrogen atoms and/or when $R_3$ in intermediates is an alkyl group bearing a hydroxy substituent and/or when $R_7$ in certain intermediates is an alkyl group bearing a hydroxy or primary or secondary amino substituent and/or when $R_5$ in intermediates is a hydrogen atom. Standard protection and deprotectin procedures can be employed. For example an amino group may be protected by formation of a phthalimide which may subsequently be cleaved by treatment with a hydrazine e.g. hydrazine hydrate or a primary amine, for example methylamine; or by formation of a benzyl derivative which may subsequently be cleaved by hydrogenolysis in the presence of a catalyst e.g. palladium. When A and B both represent N and $R_3$ is hydrogen, this may be protected by formation of a N-alkoxyalkyl (e.g. ethoxymethy) derivative which may subsequently be cleaved by treatment with dilute acid. The hydroxyl group $OR_5$ where $R_5$ is hydrogen may be protected, for example as an acyloxy group or as an ether group such as trialkylsilyl e.g. trimethylsilyl, aralkyl such as benzyl, benzhydryl or trityl, tetrahydropyranyl or alkoxymethyl, e.g. methoxymethyl ethers. Such protecting groups may be removed by conventional procedures of JFW McOmine, For example, benzyl and benzyhydryl ether groups may be removed by catalytic hydrogenolysis with for example hydrogen and palladium catalyst, and trityl, tetrahydropyranyl, alkoxymethyl and trialkylsilyl ether groups may be removed by acid hydrolysis.

In describing the process which may be used for preparing the compounds of formula (I) or intermediates useful in the preparation thereof, any of $R_1$ to $R_{24}$, Alk, Q, Z, p, n and m are as defined in formula (I) unless otherwise stated.

Compounds of formula (I) may be prepared by cyclisation of an appropriate intermediate. Thus, compounds of formula (I) in which $R_5$ represents hydrogen and Z represents the group

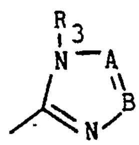

where either A represents $CR_7$ and B represents N or A represents N and B represents $CR_7$, and $R_7$ is other than acyloxyalkyl, alkoxy, nitro, $(CH_2)_qH{=}CR_{15}R_{16}$, $SO_2R_{17}$, $COR_{20}$ (where $R_{20}$ is hydrogen, aryl or aralkyl) or $CR_{23}{=}NR_{24}$, or when A represents $CR_7$ and B represents N then $R_3$ and $R_7$ may not represent the group $-(CH{=}CH)_2-$ or $-(CH_2)_4-$ may be represented by cyclising a compound of formula (III)

$$R_1R_2NAlkQO(CH_2)_n\overset{\overset{OR_5}{|}}{C}H(CH_2)_mNH\underset{\underset{V'}{\|}}{C}-\overset{\overset{R_{25}}{|}}{N}-NHY' \qquad \text{(III)}$$

in which $R_{25}$ is as defined for $R_3'$, V' is

$$\overset{NCR_7'}{\underset{V}{\|}}$$

or NCN and Y' is hydrogen where V is oxygen or sulphur and $R_7'$ is a group as defined for $R_7$ or a group convertible thereto under the conditions of the cyclisation reaction or $R_7'$ represents halogen or alkoxy; or V' is $NR_3$, $R_{25}$ is hydrogen and Y' is

$$\overset{CR_7}{\underset{Y''}{\|}}$$

where Y'' is sulphur, oxygen or NH.

Thus for example in one embodiment of the cyclisation process a compound of formula (I) in which A is N and B is the group $CR_7$ may be prepared by cyclisation of a compound of formula (IV)

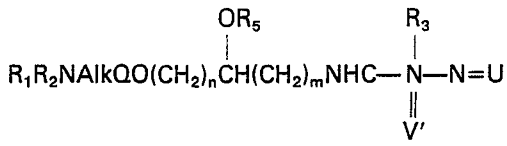

(IV)

in which V′ is NCN or NC(=V)$R_7$′ where $R_7$′ is as defined above, V represents sulphur or more preferably oxygen and U represents two hydrogen atoms, in the absence or presence of a solvent, e.g. acetone or water, and optionally with heating.

It may be convenient to prepare *in situ* compounds of formula (IV) in which U represents two hydrogen atoms by treating a compound of formula (IV) where U represents a divalent protecting group which can readily be removed to yield two hydrogen atoms, for example a benzylidene group, with an acid, e.g. hydrochloric acid, optionally in the presence of an additional solvent. e.g. toluene and conveniently at a temperature of 10—50°C. Under such conditions cyclisation to give the corresponding compound of formula (I) will normally occur.

In general the intermediate of formula (IV) may be prepared from the appropriate diamines by methods analogous to those described in British Patent Specifications Numbers 2047238A, 2023133A and 2075007A.

In another embodiment of the cyclisation process; compounds of formula (I) in which A is the group $CR_7$ and B is N may be prepared by cyclisation of a compound of formula (V)

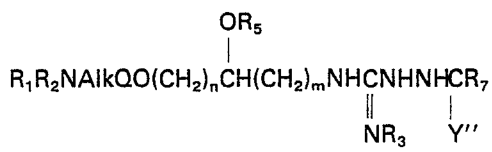

(V)

in which Y′′ is oxygen.

The cyclisation may be carried out in the absence or presence of a solvent (e.g. dimethylformamide), at elevated temperatures (e.g. within the range 80—150°C) optionally in the presence of a base e.g. aqueous potassium hydroxide.

In a convenient embodiment of the cyclisation process the intermediate (V) may be formed *in situ* by reacting an aminoguanidine of formula (VI)

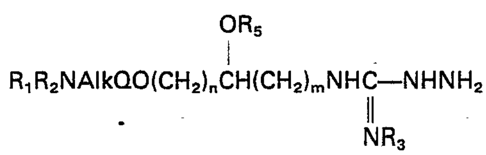

(VI)

with cyanogen bromide or with an acid $R_7CO_2H$ or and activated derivative thereof, such as an acid halide (e.g. $R_7COCl$) or a trialkylorthoester (e.g. $R_7C(OEt)_3$). The reaction may be carried out by heating the acid and the aminoguanidine (VI) under which conditions cyclisation of the intermediate (V) takes place directly to give a compound of formula (I). In the case of an activated derivative an oprotic solvent, e.g. tetrahydrofuran may be used at temperatures from ambient to reflux. Wdhen using an acyl chloride as the activated derivative the reaction may also be carried out in the presence of a base, e.g. a tertiary amine such as pyridine, which may also be used as a solvent. The reaction with cyanogen bromide may be carried out with heating, optionally in the presence of a solvent such as an alkanol e.g. methanol.

The aminoguanidine (VI) may be prepared, for example by reacting a diamine of formula (VII)

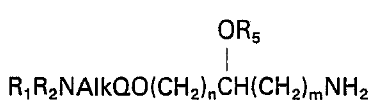

with a compound of formula (VIII)

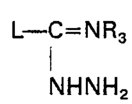

where L is a leaving group such as thioalkyl, e.g. thiomethyl.

Compounds of formula (I) in which $R_5$ is hydrogen and Z is as defined in formula (I) except $C(=X)NHR_8$ and except that when A or B represents the group $CR_7$ then $R_7$ represents nitro or when A represents $CR_7$ then $R_3$ and $R_7$ together represents the group $-(CH=CH)_2-$, may be prepared by heating the diamine (VII) in which $R_5$ represents hydrogen with a compound of formula (IX) or (X)

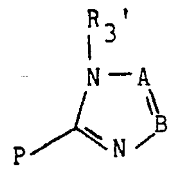

(IX)

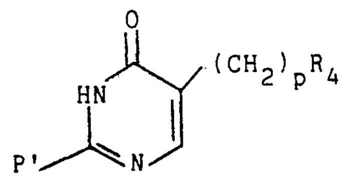

(X)

in which $R_3'$ is the group $R_3$ or a group convertible thereto, and P and P′ are leaving groups. Examples of the leaving group P are halogen e.g. bromine and P′ are nitroamino and alkylithio e.g. methylthio.

The reaction may be carried out in the absence or presence of a solvent such as acetonitrile, water or an alcohol (e.g. ethanol) at for example 80—150°C, and optionally in a sealed vessel.

In a particular embodiment of the process, triazoles in which $R_7$ is $NO_2$, i.e. q is zero and $R_6$ is $NO_2$, may be prepared by heating a diamine of formula (VII) with a triazole of formula (IX) in which $R_7$ is $NO_2$.

Compounds of formula (IX) in which A is N and B is $CR_7$ or in which A is $CR_7$ and B is N and P is bromine may be prepared from the corresponding triazole (IX) in which P is hydrogen by treatment with bromine. Compounds of formula (IX) in which A and B are both N are either known compounds or may be prepared by methods analogous to those described in British Patent Specification No. 1364917 and G. B. Barlin, J. Chem. Soc. (B) *1967*, 641.

Compounds of formula (I) where Z represents

$$-\underset{\underset{X}{\|}}{C}NHR_8$$

and $R_5$ represents hydrogen, may be made by reacting an amine of the formula (XI)

$$R_{26}NH_2 \qquad (XI)$$

with a compound of general formula (XII)

$$R_{27}NH\underset{\underset{X}{\|}}{C}-L \qquad (XII)$$

wherein one of the groups $R_{26}$ and $R_{27}$ represents the group

$$R_1R_2NAlkQO(CH_2)_n\overset{\overset{OR_5}{|}}{C}H(CH_2)_m$$

and the other represents the group $R_8$, and L is a leaving group such as halogen, thiolkyl (preferably thiomethyl) or alkoxy.

Compounds of formula (XII) may be prepared by reacting the amine (XI) with a compound of formula (XIII)

$$L-\underset{\underset{X}{\|}}{C}-L \qquad (XIII)$$

where L is as defined in formula (XII).

The above reaction may be effected in the absence of presence of a solvent e.g. ethanol or water at a

temperature from ambient to reflux preferably at room temperature. In the absence of a solvent the reaction may be carried out by heating a mixture of the reactants at for example 100—120°C.

Compounds of formula (I) in which $R_5$ represents hydrogen, A represents N and B represents $CR_7$ or A represents $CR_7$ and B represents N, where $R_7$ is the group $[CH_2]_qR_6$ where $R_6$ is $NR_{10}COR_{13}$, $NR_{10}SO_2R_{12}$, $CH_2NHC(=X)NHR_9$ or $N=CR_{15}R_{16}$ may be prepared by treating an aminoalkyltriazole of formula (XIV)

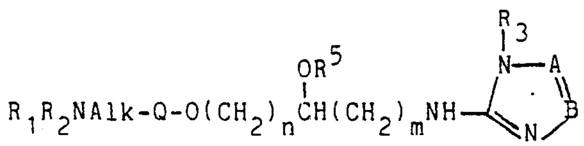

(XIV)

in which $R_1$, $R_2$ and $R_3$ are as defined in formula (I) or are groups readily convertible thereto, A represents N and B represents $CR_{28}$ or A represents $CR_{28}$ and B represents N, where $R_{28}$ is the group $(CH_2)_qNHR_{10}$, the group $(CH_2)_{q+1}NH_2$ or the group $(CH_2)_qNH_2$, with a compound capable of replacing the hydrogen atom in the group $NHR_{10}$ by the group $COR_{13}$ or $SO_2R_{12}$ or a hydrogen atom in the group $NH_2$ of the group $(CH_2)_{q+1}NH_2$ by the group $C(=X)NHR_9$ or both hydrogen atoms in the group $NH_2$ of the group $(CH_2)_qNH_2$ by the group $=CR_{15}R_{16}$.

Thus for example the aminoalkyltriazole (XIV) in which $R_{28}$ is the group $(CH_2)_q$ $NHR_{10}$ may be reacted with an isocyanate $R_{14}'NCO$ in which $R_{14}$ has any of the meanings defined for $R_{14}$ in formula (I) except hydrogen or represents an alkali metal atom such as potassium or sodium, or with an activated derivative of either a carboxylic acid $R_{13}COOH$ (in which $R_{13}$ is other than the group $NHR_{14}$) or a sulphonic acid $R_{12}SO_3H$ to give a compound of formula (I) in which $R_6$ is respectively the group $NR_{10}CONHR_{14}$, $NR_{10}COR_{13}$ (in which $R_{13}$ is other than $NHR_{14}$), or $NR_{10}SO_2R_{12}$.

Suitable activated derivatives include acid halides e.g. acid chlorides, alkylchloroformates, acid anhydrides including mixed anhydrides (e.g. acetic formic anhydride), esters such as alkyl esters, ortho esters and (1-alkyl-2-pyridinyl) esters.

The reaction with an acid halide is preferably carried out in the presence of a base e.g. an inorganic base such as sodium hydroxide or an organic base such as triethylamine or pyridine. The reaction with an alkylchloroformate is preferably carried out in the presence of a base, e.g. potassium carbonate or triethylamine, in a solvent such as dimethylformamide. The reaction with an acid anhydride may be carried out in the absence or presence of solvent such as pyridine.

In the reaction with an isocyanate, compounds of formula (I) in which $R_{14}$ is other than hydrogen are conveniently prepared by carrying out the reaction in a solvent such as acetonitrile at temperatures from ambient to reflux. Compounds of formula (I) in which $R_{14}$ is hydrogen may be prepared by heating a salt e.g. hydrochloride of the aminotriazole (XIV) with an aqueous solution of an appropriate cyanate, e.g. potassium cyanate.

As a further embodiment of this process and aminoalkyltriazole (XIV) in which $R_{28}$ is the group $(CH_2)_{q+1}NH_2$ may be treated with a compound of formula $L'C(=X)NHR_9$. The reactants may for example be mixed in an aqueous solution at room temperature.

In yet another embodiment of this process an aminoalkyltriazole (XIV) in which $R_{28}$ is the group $(CH_2)_qNH_2$ is treated with an appropriate aromatic aldehyde, e.g. benzaldehyde, or a ketone $R_{15}R_{16}CO$ to give a product in wich $R_6$ is $N=CR_{15}R_{16}$. The reaction may conveniently be carried out in the presence of a solvent e.g. benzene, preferably with heating e.g. at reflux.

Compounds of formula (I) in which $R_5$ is hydrogen and $R_7$ is the group $(CH_2)_qR_6$ in which $R_6$ is $COR_{20}$ (where $R_{20}$ is hydrogen, alkyl, aryl or aralkyl), or $SO_2R_{17}$ may be prepared by oxidation of the corresponding compound in which $R_7$ is the group $(CH_2)_qCHR_{20}OH$, $(CH_2)_qSR_{17}$ (where $R_{17}$ is other than hydroxy) or $(CH_2)_qSH$.

Thus aldehydes and ketones of formula (I) in which $R_5$ is hydrogen and $R_7$ is the group $(CH_2)_qCOR_{20}$ where $R_{20}$ is hydrogen, alkyl, aryl or aralkyl may be prepared by oxidising the corresponding hydroxyalkyl compound in which $R_7$ is $(CH_2)_qCHR_{20}OH$ using for example oxalyl chloride and dimethylsulphoxide, or activated manganese dioxide in a solvent such as dichloromethane.

Compounds of formula (I) in which $R_5$ is hydrogen and $R_7$ is the group $(CH_2)_qSO_2R_{17}$ may be prepared by oxidising the coresponding compound in which $R_7$ is either $—(CH_2)_qSR_{17}$ (where $R_{17}$ is other than hydroxy) or $(CH_2)_qSH$ with for example peractic acid. The reaction may be carried out in a solvent such as acetic acid, at room temperature.

The starting materials in which $R_7$ is $(CH_2)_qSH$ where q is other than zero may be obtained by alkaline hydrolysis of the corresponding isothiourea, which may in turn be prepared by alkylating thiourea with an appropriate compound of formula (I) in which $R_6$ is a leaving group e.g. halo.

The thiol starting materials in which $R_7$ is SH may be prepared by diazotisation of the corresponding aminotriazole followed by treatment with an alkali metal (e.g. potassium) salt of ethyl xanthate to give a xanthate in which $R_7$ is the group $—SC(=S)OEt$, which is subsequently hydrolysed (for example by heating with ethanolic potassium hydroxide) to give the starting thiol in which $R_7$ is the group SH.

The above oxidation process is particularly applicable to the preparation of compounds of formula (I)

in which there is no unsaturation within the groups $R_1$ and $R_3$.

Compounds of formula (I) in which $R_5$ is hydrogen and $R_7$ is $(CH_2)_qCR_{23}{=}NR_{24}$ may be prepared by reacting the corresponding carbonyl compound i.e. a compound of formula (I) in which $R_7$ is $(CH_2)_qCOR_{23}$, with an appropriate reagent $H_2NR_{24}$ in a suitable solvent such as ethanol optionally with heating.

Compounds of formula (I) in which $R_5$ is hydrogen, $R_7$ is $(CH_2)_qR_6$ where $R_6$ is $SO_2NR_{18}R_{19}$ or $CONR_{21}R_{22}$ may be prepared by reacting an activated derivative of the corresponding carboxylic acid or sulphonic acid, i.e. compounds of formula (I) in which $R_7$ is $(CH_2)_qR_6$ where $R_6$ is $CO_2H$ or $SO_3H$, with ammonia or an appropriate amine $HNR_{18}R_{19}$ or $HNR_{21}R_{22}$. Suitable activated derivatives include those referred to previously e.g. acid chlorides and esters.

Compounds of formula (I) in which $R_7$ is an acyloxyalkyl group and/or $R_5$ is acyl may be prepared by treating the corresponding compound of formula (I) in which $R_7$ is a hydroxyalkyl group and/or $R_5$ represents hydrogen with either an appropriate acid or an activated derivative thereof. The reaction may be carried out at room temperature optionally in the presence of a solvent (e.g. pyridine, triethylamine or an alkali metal carbonate such as potassium carbonate).

Compounds of formula (I) in which $R_5$ is hydrogen and A is $CR_7$ and B is N, and $R_3$ and $RF_7$ together represent $-(CH_2)_4-$ may be prepared by reduction of the corresponding compound in which $R_3$ and $R_7$ together represent $\{-CH{=}CH-\}_2$ using for example hydrogen and a metal catalyst (e.g. platinum) in a solvent such as ethanol.

Compounds of the formula (I) in which Z is

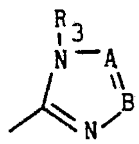

Alk is $CH_2$ and $R_5$ is hydrogen may be prepared by treating an aldehyde of formula (XV)

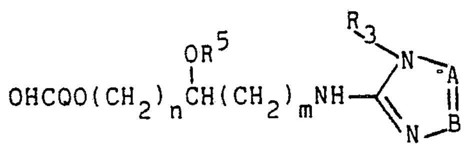

(XV)

with an amine $R_1R_2NH$ in a solvent such as tetrahydrofuran or an alkanol, e.g. methanol, followed by reduction using for example a hydride reducing agent such as an alkali or alkaline earth metal borohydride e.g. sodium borohydride or lithium aluminium hydride, or hydrogen and a metal catalyst such as palladium or platinum. The reactions may be carried out at a temperature of 0°C to 30·C.

The intermediates of formula (XV) may be prepared from compounds of formula (XVI)

$$WQO(CH_2)_nCH(OR_5)(CH_2)_mNH_2 \qquad (XVI)$$

in which W represents a protected aldehyde group, e.g. a cyclic acetal such as an ethylene acetal, by methods analogous to those described herein for preparing compounds of formula (I) from the amine of formula (VII).

Compounds of formula (I) in which $R_5$ is hydrogen and $R_6$ is tetrahydropyranyloxy may be prepared by reacting the corresponding hydroxyalkyltriazole of formula (I) with dihydropyran. The reaction may be carried out in a solvent, e.g. dichloromethane or dimethylformamide, at low temperature e.g. −10° to 0°C, in the presence of a catalyst, e.g. paratoluenesulphonic acid.

In the above discussion of the processes available for the production of compounds according to the invention reference has been made to the primary amines of formula (VII). These amines are novel compounds and the invention includes such compounds. These intermediates may be made by a number of processes which are described below.

Diamines of formula (VII) in which $R_5$ is hydrogen and n is 1 may be prepared by reacting a compound of formula (XVII)

$$R_1R_2NAlkQOH \qquad (XVII)$$

with an epoxide of formula (XVIII)

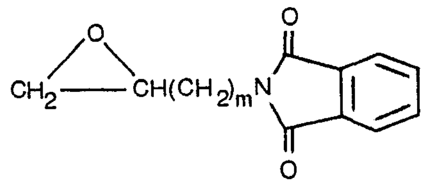

(XVIII)

to produce a diamine of formula (XIX)

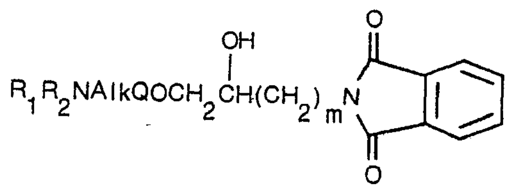

(XIX)

The reaction may be carried out in the absence or presence of a solvent such as dimethylformamide, preferably at elevated temperature, and optionally in the presence of a base, e.g. sodium hydride or potassium butoxide. The protecting group may be removed from the compound of formula (XIX) by reaction with a hydrazine, e.g. hydrazine hydrate, or a primary amine, e.g. methylamine.

Diamines of formula (VII) in which $R_5$ is hydrogen and m is 1 may be prepared by reacting an epoxide of formula (XX)

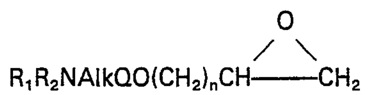

(XX)

with an azide, e.g sodium azide to produce a compound of formula (XXI)

OH

$$R_1R_2NAlkQO(CH_2)_nCHCH_2N_3 \qquad (XXI)$$

which may be reduced to produce a diamine of formula (VII) where im is 1. The reaction with the azide may be carried out in a suitable solvent, e.g. aqueous ethanol in the presence of ammonium chloride, preferably at reflux temperature. Reduction of the compound of formula (XXI) may be carried out for example, with lithium aluminium hydride in a suitable solvent, e.g. tetrahydrofuran, or catalytically using for example platinum oxide of palladium oxide as catalyst.

Diamines of formula (VII) in which $R_5$ is hydrogen and m is 2 may be prepared by reacting the epoxide of formula (XX) with a cyanide, e.g. sodium cyanide, to produce a compound of formula (XXII)

OH

$$R_1R_2NAlkQO(CH_2)CHCH_2CN \qquad (XXIII)$$

which may produce a compound of formula (VII) in which m is 2. The reaction with the cyanide may be carried out in a suitable solvent, e.g. aqueous ethanol preferably at reflux temperature. Reduction of the compound of formula (XXII) may be carried out, for example with lithium aluminium hydride in a suitable solvent, e.g. tetrahydrofuran. Alternatively the epoxide of formula (XX) may be reacted with nitromethane to produce a compound of formula (XXIII)

OH

$$R_1R_2NAlkQO(CH_2)_nCHCH_2CH_2NO_2 \qquad (XXII)$$

which may be reduced to produce a compound of formula (VII) in which m is 2. The reaction with nitromethane may be carried out in a suitable solvent, e.g. dimethylformamide, preferably in the presence of a base, e.g. sodium hydride. Reduction of the compound of formula (XXIII) may be carried out for example as described above for reduction of the compound of formula (XXII) or using hydrogen in the presence of a catalyst.

The intermediate epoxides of formula (XX) may be prepared by alkylation of an appropriate alkali metal phenolate, e.g. sodium phenolate with a halohydrin (XXIV)

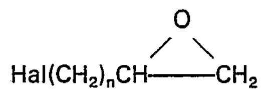

(XXIV)

The intermediate epoxide of formula (XVIII) may be prepared by alkylation of an alkali metal, phthalimide, e.g. potassium phthalimide with a halohydrin (XXV)

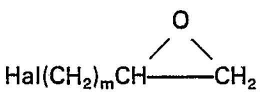

(XXV)

Where the product of any of the above processes is a free base and a salt is required, the salt may be formed in conventional manner. Thus, for example, a generally convenient method of forming the salts is to mix appropriate quantities of the free base and the acid in an appropriate solvent(s) e.g. an alcohol such as ethanol or an ester such as ethyl acetate.

The invention is illustrated but not limited by the following Examples.

In the following Examples and Preparations temperatures are in °C.

T.l.c. refers to thin layer chromatography and this and preparative chromatography were carried out on silica using, unless otherwise stated, one of the following solvent systems.

System A: Dichloromethane:ethanol:0.88 ammonia (50:8:1)
System B: Dichloromethane:ethanol:0.88 ammonia (100:8:1)
System C: Methanol:0.88 ammonia (200:1)

Preparation 1
2-[2-Hydroxy-3-[3-(1-piperidinylmethyl)phenoxy]propyl]-1H-isoindole-1,3-(2H)dione

A mixture of 2-(oxiranylmethyl)-1H-isoindole-1,3-(2H)-dione (9.10 g) and 3-(1-piperidinylmethyl)phenol (8.55 g) was heated at 130°C under nitrogen for 10 minutes. The resulting mixture was dissolved in chloroformm (100 ml), washed with 1H sodium hydroxide (2 × 25ml), dried ($MgSO_4$) and evaporated to give the title compound as a gum (17.65 g).

T.l.c. system B, Rf 0.60.

b) Similarly prepared by this procedure from 2-[2-oxiranylethyl]-1H-isoindole-1,3-(2H)dione (19.7 g) and 3-(1-piperidinylmethyl)phenol (17.4 g) except that the crude compound was distilled, was 2-[3-hydroxy-4-[3-(1-piperidinylmethyl)phenoxy)butyl]-1H-isoindole-1,3-(2H)-dione (16.5 g) as an orange oil, b.p. 180·C (0.05 m)

N.m.r. ($CDCl_3$): 2.0—2.35, m, (4H); 2.8, t, (dd), (1H); 3.33, m, (3H); 5.8—6.2, m, (5H); 6.54, s, (2H); 6.95, br, (1H), 7.5—7.7, m, (4H); 7.8—8.2, m, (2H); 8.2—8.7, m, (6H).

Preparation 2
1-Amino-3-[3-[(1-piperidinylmethyl)phenoxy]-2-propanol

A solution of 2-[2-hydroxy-3-[3-(1-piperidinylmethyl)phenoxy]propyl]-1H-isoindole-1,3-(2H)dione (17.6 g) and hydrazine hydrate (2.5 g) in ethanol (60 ml) was heated under reflux for 3h. The resulting mixture was evaporated to a solid residue which was suspended in 1N hydrochloric acid (30 ml) and filtered. The filtrate was basified with an excess of potassium carbonate and extracted with isopropanol (3 × 40 ml). The isopropanol extracts were dried ($Na_2CO_3$) and evaporated to gum which was chromatographed using System A. Crystallisation of the product from n-hexane:ether (20:l) gave the *title compound* as colourless grains (7.7 g), m.p. 74—76.5°.

b) Similarly prepared by this procedure from 2-[3-hydroxy-4-[3-piperidinylmethyl)phenoxy]butyl]-1H-isoindole-1,3-(2H)-dione (16.5 g) and hydrazine hydrate (4.35 g) (except that the crude product was distilled [b.p. 200° (0.06 mml)]) was 4-amino-1-[3-(1-piperidinylmethyl)phenoxy)butyl]-2-butanol (7.2 g) as an white solid, m.p. 59°.

c) Similarly prepared by this procedure from 2-[3-[3-(dimethylaminomethyl)-2-hydroxy-propyl]-1*H*-isoindole-1,3-(2H)-dione (13.0 g) and hydrazine hydrate (2.1 g) except the crude product was distilled [b.p. 200°, 0.2 mmHg] was 1-amino-3-[3-(dimethylaminomethyl)phenoxy]-2-propanol (3.8 g) as a pale yellow solid.

n.m.r. ($CDCl_3$): 2.7, t, (1H); *ca.* 3.2, m, (3H); *ca.* 6.05, m, (3H); 6.63, s, (2H); 7.15, m, (2H); 7.53, br, m, (2H); 7.78, s, (6H); 7.0—8.3, br, s, (1H).

Preparation 3
2-[3-[3-(Dimethylaminomethyl)phenoxy]-2-hydroxy-propyl]-1H-isoindole-1,3-(2H)dione

A solution of 2-(oxiranylmethyl)-1*H*-isoindole-1,3-(2H)-dione (20.3 g) and 3-(dimethylaminomethyl)phenol (22.3 g) in dimethylformamide (200 ml) with a catalytic amount of sodium hydride (0.2 g) was heated for 6 h at 100° under $N_2$. The solvent was evaporated to give an oil which was dissolved in chloroform and washed with 2N sodium hydroxide and water. The organic solution was

evaporated to give an oil (14 g). A portion of the oil (0.8 g) was distilled to give the *title compound* (0.6 g) as an oil, b.p. 250° (0.08 mmHg).

| | | | |
|---|---|---|---|
| Found: | C, 67,4; | H, 6.3; | N, 7.8; |
| $C_{F0}H_{22}H_2O_4$ requires: | C, 67.8; | H, 6.3; | N, 7.9% |

Preparation 4

5-Bromo-1-ethyl-1H-tetrazole

A solution of bromine (3.34 g) in chloroform (2 ml) was added dropwise over 0.25 h to a refluxing solution of 1-ethyl-1H-tetrazole (980 mg) in acetic acid (8 ml) and chloroform (16 ml). The mixture was refluxed for 16 h, was cooled and evaporated to give an oil which was distilled to give the title compound (1.28 g) as an orange oil b.p. 100—110° (0.08 mm).

n.m.r. ($CDCl_3$): 5.53, q, (2H); 8.40, t, (3H).

Preparation 5

1-Amino-4-[3-(1-piperidinylmethyl)phenoxy]-2-butanol

(a) 1-[[3-(2-Oxiranylethoxy)phenyl]methyl]piperidine

A mixture of 3-(1-piperidinylmethyl)phenol (19.1 g) and flaked potassium hydroxide (6.1 g) in acetonitrile (350 ml) was stirred at room temperature for 16 h. The mixture was warmed to give a uniform solution to which (2-bromoethyl)oxirane (20 g) was added. After 3.5 h at ambient temperature, the solvent was evaporated off and the residue partitioned between diethyl ether (400 ml) and water (100 ml). The organic phase was washed with 1M sodium hydroxide, dried and evaporated at 50° (0.01 mm) to give the *title compound* (18 g) as a light brown oil.

n.m.r. ($CDCl_3$): 2.7—3.4, t + m, (4H); 5.93, t, (2H); 6.6, s, (2H); 6.9, m, (1H); 7.23 + 7.47, t + dd, (2H); *ca.* 7.65, m, (4H); *ca.* 8.0, m, (2H); *ca.* 8.6, m, (6H).

(b) 1-Azido-4-(3-(1-piperidinylmethyl)phenoxy]-2-butanol

A solution of 1-[[3-(2-oxiranylethoxy]phenyl)methyl]piperidine (17 g), sodium azide (5.1 g) and ammonium chloride (2.73 g) in 25% aqueous ethanol (200 ml) was refluxed for 6 h and concentrated to *ca.* 130 ml. The concentrate was diluted with water (50 ml), saturated with potassium carbonate and extracted with isopropanol. The extract was dried and evaporated to give an oil (20 g) which was chromatographed on silica using dichloromethane:ethanol:ammonia (200:8:1) to give the *title compound* (10.5 g) as a colourless oil.

n.m.r. ($CDCl_3$): 2.75, t, (1H); 3.0—3.3, m, (3H); *ca.* 5.85, m, (3H); 6.53, s, (2H); 6.58, d, (2H); 7.2, br, s, (1H); 7.65, m, (4H); 8.05, m, (2H); *ca.* 8.5, m, (6H).

(c) 1-Amino-4-[3-(1-piperidinylmethyl)phenoxy]-2-butanol

A solution of 1-azido-4[3-(1-piperidinylmethyl)phenoxy)-2-butanol (10 g), in dry tetrahydrofuran (100 ml) was added dropwise to a stirred suspension of lithium aluminium hydride (4 g) in tetrahydrofuran (200 ml) under nitrogen. The resulting mixture was stirred at room temperature for 1 h and quenched with water (4 ml), followed by 15% sodium hydroxide (4 ml) and water (12 ml). The mixture was then filtered and the residue washed with tetrahydrofuran (50 ml). The combined filtrate was evaporated to give the *title compound* (7.5 g) as a pale pink oil.

n.m.r. ($CDCl_3$): 2.78, t, (1H); 3.0—3.32, m, (3H); 5.88, t, (2H); 6.22, m, (1H); 6.58, s, (2H); 7.0—7.5, *ABX*, (2H); 7.6, m, (4H); 7.73, brs, (3H); 8.1, m, (2h); *ca.* 8.5, m, (6H).

Example 1

1-[(3-Amino-1-methyl-1H-1,2,4-triazol-5-yl)amino-3-[3-(1-piperidinylmethyl)phenoxy)-2-propanol, maleate (1:2)

A mixture of 1-amino-3-[(1-piperidinylmethyl)phenoxy)-2-propanol (1.32 g) and methyl N-cyano-1-methyl-2-(phenylmethylene)hydrazine-carboximidothioate (1.16 g) was heated as a melt at 80°C under water pump vacuum until bubbling ceased (20 minutes). The resulting mixture was stirred with toluene (20 ml) and 1N hydrochloric acid (10 ml) for 30 minutes. The aqueous phase was diluted with water (30 ml), brought to pH 9 by addition of potassium carbonate, and washed with toluene (2 × 40 ml). The aqueous layer was treated with an excess of potassium carbonate and extracted with ethyl acetate (3 × 25 ml). The ethyl acetate extracts were dried ($Na_2SO_4$) and evaporated to a gum which was purified by column chromatography using System A. The resulting gum was treated with an excess of maleic acid in isopropanol to yield a title compound as a white solid (1.52 g), m.p. 145—7°

| | | | |
|---|---|---|---|
| Found: | C, 52,4; | H, 6.1; | N, 13.9; |
| $C_{26}H_{36}H_6O_{10}$ requires: | C, 52.9; | H, 5.8; | N, 14.2% |

Example 2

5-[[2-hydroxy-3-[3-(1-piperidinylmethyl)phenoxy]propyl] amino]-1-methyl-1H-1,2,4-triazole-3-methanol

A solution of 1-amino-3-[(1-piperidinylmethyl)phenoxy]-2-propanol (compound A) (1.23 g) and methyl

N-[2-(acetyloxy)acetyl]-1-methyl-2-(phenylmethylene)-hydrazinecarboximidothioate (Compound B) (1.61 g) in toluene was stirred at 25°C for 4 h. The resulting solution was treated with 5N hydrochloric acid and stirred for 18 h. The aqueous layer was adjusted to pH 9 by addition of sodium hydroxide and washed with toluene. The aqueous layer was treated with an axcess of sodium hydroxide and extracted with hot methyl isobutyl ketone which on cooling gave a white precipitate. Recrystallisation from isopropanol gave the *title compound* as a white solid (0.81 g) m.p. 170—2°

| | | | |
|---|---|---|---|
| Analysis Found: | C, 60.8; | H, 7.7; | N, 18.3 |
| $C_{19}H_{29}N_5O_3$ requires: | C, 60.8; | H, 7.8; | N, 18.7% |

b) Similarly prepared by this procedure from compound A (0.8 g) and methyl 1-methyl-N-[(methylsulphonyl)acetyl]-2-(phenylmethylene)hydrazine-carboximidothioate (1.1 g), except that the methyl isobuty ketone extract was evaporated to give a residue which was chromatographed using system A to give a gum which crystallised from methyl acetate — light petroleum (b.p. 60—80°), was 1-[(1-methyl-3-methylsulphonylmethyl-1H-triazol-5-yl)amino)-3-[3-1-piperidinylmethyl)phenoxy]-2-propanol, (0.49 g) as a white solid m.p. 68°.

N.m.r. ($CDCl_3$): 2.8, t, (1H); 2.95—3.35, m, (3H); 5.22, t, (1H); 5.78, s, (2H); 5.97, d, (2H); 5.9, m, (1H); 6.3, d, (2H); 6.43, s, (3H); 6.55, s, (2H); 6.9, s, (3H); 7.6, m, (4H); 8.45, m, (6H).

c) Similarly prepared by this procedure from 4-amino-1-[3-(1-piperidinylmethyl)phenoxy)-2-butanol-(2.0 g) and compound B (2.4 g), was 1-methyl-5-[3-hydroxy-[4-[3-(1-piperidinylmethyl)phenoxy]-butyl]amino]-1H-1,2,4-triazole-3-methanol (1.7 g) as a white solid, m.p. 128°.

| | | | |
|---|---|---|---|
| Found: | C, 61.7; | H, 8.0; | N, 17.7 |
| $C_{20}H_{31}N_5O_3$ requires: | C, 61.7; | H, 8.0; | N, 18.0% |

d) Similarly prepared by this procedure from 1-amino-3-[3-(dimethylaminomethyl)phenoxy]-2-propanol (1.5 g) and compound B (2.25 g) except that the methyl isobutyl ketone extract was evaporated to give a solid which was washed with diethyl ether and then recrystallised from methyl acetate was 5-[[3-[3-(dimethylaminomethyl)phenoxy]-2-hydroxy-propyl]amino]-1-methyl-1*H*-1,2,4-triazole-3-methanol (250 mg) m.p. 104—109°.

N.m.r. ($CD_3OD$): 2.7—3.55, m, (4H); 5.7, s, (2H); 5.8—6.3, m, (3H); 6.45—6.75, m, (7H); 7.9, s, (6H).

## Example 3

### 1-[[3-(3-Aminopropyl)-1-methyl-1*H*-1,2,4-triazol-5-yl]amino]-3-[3-(1-piperidinylmethyl)phenoxy]-2-propanol

2-[3-[5-[[2-Hydroxy-3-[3-(1-piperidinylmethyl)phenoxy]propyl]amino]-1-methyl-1*H*-1,2,4-triazol-3-yl]propyl)-1*H*-isoindole-1,3(2H)-dione

A solution of methyl-N-[4-(1,3-dihydro-1,3-dioxo-2*H*-isoindol-2-yl)-1-oxobutyl]-1-methyl-2-(phenylmethylene)hydrazinecarboximidothioate, (2.1 g) and 1-amino-3-[(1-piperidinylmethyl)phenoxy]-2-propanol (1.32 g) in toluene (10 ml) was stirred at room temperature 6 h. The mixture was then treated with 5N hydrochloric acid (10 ml) at room temperature for 21h and a further quantity of 5N hydrochloric acid (5 ml) was added and stirred for further 20 h. The reaction mixture was then washed with toluene, basified with sodium bi-carbonate (pH 9) and extracted with ethyl acetate. The extract was dried and evaporated to leave a light brown gum (1.9 g) which was chromatographed using System A to give the *title compound* (0.8 g) as a yellow gum.

n.m.r. ($CDCl_3$): 2.2, m, (4H); 2.84, t, (1H); 3—3.35, m, (3H); 5.25, t, (1H); 4.6—5.8, br, (1H); 5.7—6.8, m, (12H); 7.25—8.15, m, (8H); 8.2—8.7, m, (6H).

1-[[3-(3-Aminopropyl)-1-methyl-1H-1,2,4-triazol-5-yl]amino]-3-[3-(1-piperidinylmethyl)phenoxy]-2-propanol

A solution of 2-[3-[5-[[2-hydroxy-3-[3-(1-piperidinylmethyl)phenoxy]propyl]amino]-1-methyl-1*H*-1,2,4-triazol-3-yl]propyl]-1*H*-isoindole-1,3(2*H*)-dione (0.8 g) and hydrazine hydrate (0.5 ml), in ethanol (60 ml) was stirred at room temperature for 4 h and the solvent removed *in vacuo.* The white solid residue was partitioned between diethyl ether (100 ml) and 5M sodium hydroxide (50 ml). The alkaline aqueous phase was further extracted with ethyl acetate. The organic extracts were combined, washed with 5M sodium hydroxide (50 ml), dried and evaporated to give a yellow gum (0.65 g) which was chromatographed on silica using methanol: 0.88 ammonia (80:.1) to give the *title compound* (0.25 g) as a light brown gum.

n.m.r. ($CDCl_3$): 2.8, dd (t), (1H); 3.01—3.33, m, (3H); 5.37, t, (1H); 5.8—6.16, m, (3H); 6.3—6.62, m, (6H); 7.05—7.8, m, (12H); 8.2—8.72, m, (8H).

i.r. ($CHCl_3$): 3600, 3445, 2800, 2755, 2720, 1595cm$^{-1}$.

## Example 4

### 5-[2-acetyloxy-[3-[3-(1-piperidinylmethyl)phenoxy]propyl]amino]-1-methyl)-1H-1,2,4-triazole-3-methanol acetate (ester)

A solution of 5-[2-hydroxy-[3-[3-(1-piperidinylmethyl)phenoxy]propyl]amino]-1-methyl-1H-1,2,4-triazole-3-methanol (0.75 g) in acetic acid (30 ml) was refluxed for 48 h. The solution was evaporated to leave a pale yellow oil. Aqueous sodium bicarbonate solution (2M, 100 ml) was added and the mixture was

extracted with ethyl acetate. The extract was dried and evaporated to give the *title compound* (0.89 g) as a yellow gum.

N.m.r. ($CDCl_3$): 2.78, t, (dd), (1H); 3.0—3.35, m, (3H); 4.65, m, (1H); 5.03, s, (2H); 5.51, t, (1H); 5.82, d, (2H); 6.1—6.3, m, (2H); 6.45, s, (3H); 6.58, s, (2H); 7.5—7.7, m, (4H); 7.90, s, (6H); 8.3—8.7, m, (6H).

I.r. ($CHBr_3$): 3440, 1740, 2800, 2760, 2720cm$^{-1}$.

### Example 5

1-[(3-Amino-1H-1,2,4-tiazol-5-yl)amino]-3-[3-(1-piperidinylmethyl)phenoxy]-2-propanol hemihydrate ·

Methyl N-cyano-[2-hydroxy-3-[3-(1-piperidinyl)methyl]phenoxy]propyl)carbamimidothioate

A solution of 1-amino-3-[(1-piperidinylmethyl)phenoxy]-2-propanol (2.6 g) in ether (50 ml) and methanol (10 ml) was added dropwise to a solution of dimethyl cyanocarbonimidodithioic acid (1.5 g) in ether (30 ml) and the mixture was stirred at room temperature for 18 h. The mixture was filtered and the filtrate was evaporated to give a gum which was chromatographed using System B to give the title compound (1.32 g) as a pale yellow gum.

N.m.r. ($CDCl_3$): 2.8, t, (1H); 2.9—3.4, m, (3H); 4.8, m, (1H); 5.8, m, (2H); 6.1, m, (2H); 6.3, s, (2H); 7.35, s, (3H); 7.6, m, (4H); *ca.* 8.5, m, (6H).

1-[(3-Amino-1H-1,2,4-triazol-5-yl)amino]-3-[3-(1-piperidinylmethyl)phenoxy]-2-propanol hemihydrate

Hydrazine hydrate (0.9 ml; 18.5 mmol) was added to a stirred solution of methyl N-cyano-[2-hydroxy-3-[3-(1-piperidinyl)methyl]phenoxy]propyl]carbamimidothioate (1.25 g, 3.5 mmol) in ethanol (10 ml). After 18 h at room temperature the mixture was evaporated to give a gum (1.3 g) which was chromatographed on silica using methanol:0.88 ammonia (100:1) to give the *title compound* (0.96 g) as a solid white foam, m.p. 57° (softens).

N.m.r. ($CD_3OD$): 2.82, t, (dd), (1H); 3.0—3.3, m, (3H); 5.8—6.15, m, (3H); 6.60, s, (2H); 6.7, m, (2H); 7.60, m, (4H); 8.3—8.7, m, (6H).

### Example 6

1-[3-(1-piperidinylmethyl)phenoxy]-3-[(1,2,4-triazolo[4,3,α]pyridin-3-yl)amino]-2-propanol

A solution of 1-amino-3-[3-(1-piperidinylmethyl)phenoxy]-2-propanol (1.6 g) and 3-bromo-1,2,4-triazolo[4,3—α]pyridine (1.2 g) in absolute ethanol (5 ml) was heated in an autoclave at 140° for 42 h. Aqueous 2M sodium carbonate (15 ml) was added to the reaction mixture and the solvent was evaporated to leave a dark brown solid. This solid was extracted with hot ethyl ecetate. The ethyl acetate extract was cooled and partitioned with dilute hydrochloric acid (1M; 50 ml). The acidic aqueous layer was basified with excess aqueous sodium carbonate, washed with diethyl ether (50 ml) and then extracted with diethyl ether. The organic extracts were combined, dried ($Na_2SO_4$) and evaporated to leave a brown semi-solid gum. This gum was triturated with dry diethyl ether to precipitate a white solid (0.75 g) which was recrystallised from a mixture of ethyl acetate and methanol to give the *title compound* (0.15 g) as a white solid m.p. 160—161°.

| | | | |
|---|---|---|---|
| Analysis Found: | C, 66.1; | H, 6.9; | N, 18.2; |
| $C_{21}H_{27}N_5O_2$ requires: | C, 66.1; | H, 7.1; | N, 18.4% |

### Example 7

1-[(1-Methyl-1H-tetrazol-5-yl)amino]-3-[3-(1-piperidinylmethyl)phenoxy]-2-propanol

A solution of 1-amino-3-[3-(1-piperidinylmethyl)-phenoxy]-2-propanol (1.19 g) and 5-bromo-1-methyl-1*H*-tetrazole (0.735 g) in absolute ethanol (6 ml) was heated in an autoclave at 125° for 18 h. The mixture was cooled, evaporated, saturated with sodium carbonate solution (30 ml) and ethyl acetate (30 ml) added. The mixture was stirred for 15 min. and filtered to give a white solid (830 mg) which was recrystallised from ethyl acetate (20 ml) to give the *title compound* (0.58 g) as white micro-crystals, m.p. 156—7°.

| | | | |
|---|---|---|---|
| Analysis Found: | C, 58.73; | H, 7.60; | N, 24.24; |
| $C_{17}H_{26}N_6O_2$ requires: | C, 58.94; | H, 7.57; | N, 24.26% |

### Example 8

2-[[2-Hydroxy-3-[3-(1-piperidinylmethyl)phenoxy]propyl]amino]-5-[(6-methyl-3-pyridinyl)methyl]-4-(3*H*)-pyrimidinone

A mixture of 5-(6-methyl-3-pyridinylmethyl)-2-methylthio-4(3*H*)-pyrimidinone (1.24 g) and 1-amino-3-[3-[1-piperidinylmethyl)phenoxy]-2-propanol (Compound A) (1.32 g) was heated at 120° for 1.5 h and then at 130—140° for 2.5 h. The resulting brown glass was dissolved in chloroform (25 ml) and added to stirred dry diethyl ether (250 ml) to precipitate a white solid (1.5 g). This solid was recrystallised from ethyl acetate to give the *title compound* (0.9 g) as a white solid, m.p. 115—117°.

N.m.r. ($CDCl_3$): 1.0—3.0, v,. br, (3H); 1.5, d, (1H); 2.53—2.59, s + dd, (2H); 2,82, t, (1H); 2.99, d, (1H); 3.10—3.3, m, (3H); 5.8—6.1. m, (3H); 6.25—6.58, ABq + s, (6H); 7.54, s, (3H); 7.65, br, (4H); 8.4—8.6, br, (6H).

Similarly prepared by this procedure were:

b) From compound A (0.9 g) and 5-(3-pyridinylmethyl)-2-methylthio-4(3H)-pyrimidinone (0.8 g), except that the mixture was heated at 125—130° for 2.5 h and subsequently at 140—150° for 3 h and the crude product (0.92 g) was chromatographed using System C was 2-[[2-hydroxy-3-[3-(1-piperidinylmethyl)-phenoxy]propyl[amino]-5-(3-pyridinylmethyl)-4(3H)-pyrimidinone monohydrate (0.42 g) as a buff solid m.p. (78—82°) (softens).

| | | | |
|---|---|---|---|
| Found: | C, 64.3: | H, 6.9: | N, 14.6: |
| $C_{25}H_{31}N_9O_3.H_2O$ requires: | C, 64.2: | H, 7.1; | N, 15.0% |

c) From compound A (0.4 g) and 5-[(4-methoxy phenyl)methyl)-2-(methylthio)-4(3*H*)-pyrimidinone (0.42 g), except that the crude product chromatographed using System C and then crystallised from methyl acetate and light petroleum (b.p. 60—80°), was 2-[[2-hydroxy-3-[3-(1-piperidinylmethyl)phenoxy]-propyl]amino]-5-[(4-methoxyphenyl)methyl]-4(3*H*-pyrimidinone (0.26 g) as a white solid m.p. 112—116°.

n.m.r. ($CDCl_3$): 2.2—3.4, m, (12H); 2.7—3.4, m, (9H); 5.95—6.15, m + d, (3H); 6.3, s, (3H); 6.6, m, (6H); 7.7, m, (4H); 8.6, m, (6H).

Example 9

1-[[1-(Methylamino)-2-nitroethenyl)amino]-3-[3-(1-piperidinylmethyl)phenoxy]-2-propanol

A solution of 1-amino-3-[(1-piperidinylmethyl)phenoxy]-2-propanol (1.32 g) and N-methyl-1-(methylthio)-2-nitroethenamine (1.04 g) in water (25 ml) was stirred at 25° for 24 h. The resulting mixture was treated with glacial acetic acid (2 ml) and was washed with ethyl acetate. The aqueous phase was basified with excess potassium carbonate and extracted with ethyl acetate. The extract was dried and evaporated to give a gum which was crystallised from acetone to give the *title compound* (820 mg) as a white solid, m.p. 104—5°.

N.m.r. ($CDCl_3$-DMSO): −0.3, br, s, (1H); 2.81, dd, (1H); 3.0—3.5, m, (4H); 5.7—6.1, m, (3H); 6.4—6.6, m, (2H); 6.6, s, (2H); 7.2, br s, (3H); 7.5—7.8, m, (4H); 8.3—8.7, m, (6H).

b) Similarly prepared by this procedure from 4-amino-1-[3-(1-piperidinylmethyl)phenoxy]-2-butanol (2.0 g) and N-methyl-1-(methylthio)-2-nitroethenamine (1.5 g) except that the gum was chromatographed using System B was 4-[[1-(methylamino)-2-nitroethenyl]amino]-1-[3-(1-piperidinylmethyl)phenoxy]-2-butanol (1.1 g) as a white foam, m.p. 45—50°

n.m.r. ($CD_3OD$): −0.2, br, (1H); 0.32, br, (1H); 2.85, t, (1H); 3.0—3.5, m + s, (4H); 5.9—6.2, m, (3H); *ca.* 6.6, m + s, (4H); 7.2, brs, (3H); 7.7, m, (4H); 8.2, m, (2H); *ca.* 8.6, m, (6H).

Example 10

1-[[3-(Acetylamino)-1-methyl-1*H*-1,2,4-triazol-5-yl]amino]-3-[3-(1-piperidinylmethyl)phenoxy]-2-propanol acetate ester

A solution of 1-[(3-amino-1-methyl-1*H*-1,2,4-triazol-3-yl]amino]-3-[3-(1-piperidinylmethyl)phenoxy]-2-propanol (1.0 g) and acetic anhydride (0.62 g) in pyridine (30 ml) was stirred at 23°C for 18 h. The solvent was evaporated to give a gum, which was dissolved in ethyl acetate. This solution was washed with saturated sodium carbonate solution, dried and evaporated to give a foam which was chromatographed using System B to give the *title compound* (0.5 g) as a white foam.

n.m.r. ($CD_3OD$): 2.7—3.3, m, (4H); 4.6, m, (1H); 5.9, m, (2H); 6.25—6.65, m, (7H); 7.6, m, (4H); 7.9—8.0, s + s, (6H).

i.r. ($CHBr_3$): 3425, 3405, 2800, 2760, 2720, 1735, 1708, 1678, 1610cm$^{-1}$.

Example 11

N-[5-[[2-Hydroxy-3-[3-(1-piperidinylmethyl)phenoxy]propyl]amino]-1-methyl-1,2,4-triazol-3-yl]acetamide hemidydrate

A solution of 1-[[3-(acetylamino)-1-methyl-1H-1,2,4-triazol-5-yl]amino]-3-[3-(1-piperidinylmethyl)-phenoxy]-2-propanol acetate (ester) (0.32 g) and 0.9M aqueous potassium hydroxide (1.0 ml) in ethanol (25 ml) was stirred for 0.5 h at 23°. The ethanol was evaporated, and the residue was suspended in sodium carbonate solution. This solution was extracted with methyl acetate. The extract was dried, and evaporated to give a gum, which was chromatographed using System A to give the *title compound* (0.25 g) as a white foam.

| | | | |
|---|---|---|---|
| Found: | C, 58.5: | H, 7.7: | N, 20.1; |
| $C_{20}H_{30}N_6O_3.5H_2O$ requires: | C, 58.4: | H, 7.6; | N,20.4% |

N.m.r. ($CDCl_3$): 2.6—3.3, m, (4H); 5.7—6.15, m, (7H); 6.4—6.6, m, (7H); 7.6, m, (4H); 7.9, s, (3H); 8.5, m, (6H).

Example 12

1-[(5,6,7,8-Tetrahydro-1,2,4-triazolo[4,3-a]pyridin-3-yl)amino]-3-[3-(1-piperidinylmethyl)phenoxy]-2-propanol

A solution of 1-[3-(1-piperidinylmethyl)phenoxy]-3-[(1,2,4-triazolo[4,3-a]pyridin-3-yl]-2-propanol (252

mg) in absolute ethanol (25 ml) was hydrogenated over a 5% platinium on carbon catalyst (150 mg). After 8 h, additional catalyst (200 mg) was added and the mixture was hydrogenated for a further 8 h. The mixture was filtered through hyflo and the filtrate was evaporated to give a white solid which was recrystallised from ethyl acetate-methanol to give the *title compound* (182 mg) as white microcrystals, m.p. 141—2°.

| | | | |
|---|---|---|---|
| Found: | C, 65.1: | H, 8.1: | N, 18.0; |
| $C_{21}H_{31}N_5O_2$ requires: | C, 65.4: | H, 8.1; | N, 18.2% |

Example 13

1-[[1-Methyl-3-[3-[[1-(methylamino)-2-nitroethenyl]amino]propyl)-1H-1,2,4-triazol-5-yl]amino)-3-[3-(1-piperidinylmethyl)phenoxy]-2-propanol

A solution of 1-[[3-(3-aminopropyl)-1-methyl-1H-1,2,4-triazol-5-yl]amino)-3-[3-(1-piperidinylmethyl)phenoxy]-2-propanol (0.25 g) and N-methyl-1-(methylthio)-2-nitroethenamine (0.1 g) in water (6 ml) and methanol (6 ml) was stirred at room temperature for 24 h. A further quantity of N-methyl-1-(methylthio)-2-nitroethenamine (0.01 g) was added and the mixture stirred for a further 3 h. The reaction mixture was concentrated to *ca.* 6 ml, diluted with water (10 ml), acidified to $pH_3$ with acetic acid and washed with ethyl ecetate. The aqueous solution was basified with excess potassium carbonate and extracted with ethyl acetate. The extract was dried and evaporated to leave a brown gum which was chromatographed on silica using methanol:ammonia (250:1) to give a gum which was dissolved in hot ethyl acetate and cooled to precipitate a gum. This gum was dissolved in chloroform and filtered through florosil and hyflo. The filtrate was evaporated to give the *title compound* (0.12 g) as a solid white foam m.p. 60—63° (softens).

n.m.r. ($CDCl_3$): −0.2, brs, (1H); 2.25, brs, (1H); 2.8, t, (1H); 3.1, m, (2H); 3.24, dd, (1H); 3.28—3.45, m, (2H); 4,4, brs, (1H); 5.75, brs, (1H); 5.95, m, (2H); 6.1—6.9, m, (9H); 7.0—7.5, m, (5H); 7.63, m, (4H); 8.05, m, (2H); 8.4—7.8, m, (6H).

Example 14

1-[[1-Methyl-3-[(phenylmethylene)amino]-1*H*-1,2,4-triazol-5-yl]amino]-3-[3-(1-piperidinylmethyl)phenoxy]-2-propanol

A solution of 1-[(3-amino-1-methyl-1*H*-1,2,4-triazol-5-yl)amino]-3-[3-(1-piperidinylmethyl)phenoxy]-2-propanol (0.47 g) and benzaldehyde (0.2 g) in benzene (15 ml) was refluxed for 18 h and evaporated to leave a brown gum (0.65 g). This gum (0.33 g) was chromatographed on alumina using diethyl ether:ethyl acetate:methanol (20:20:1) to give the *title compound* (0.09 g) as a yellow foam. m.p. 53—57° (softens).

n.m.r. ($CDCl_3$): 0.9, s, (1H); 2.1—2.6, m + m, (5H); 2.8, t, (1H); 3.0—3.35, m, (3H); 5.15, brt, (1H); 5.4—6.1, m, (4H); 6.2—6.6, m, (7H); 7.5, m, (4H); 8.5, m, (6H).

Example 15

1-[(1-Ethyl-1*H*-tetrazol-5-yl)amino]-3-[3-(1-piperidinylmethyl)phenoxy]-2-propanol

A solution of 1-amino-3-[3-(1-piperidinylmethyl)phenoxy]-2-propanol (1.06 g) and 5-bromo-1-ethyl-1*H*-tetrazole (0.7 g) in water (20 ml) was stirred at reflux for 18 h. Excess potassium carbonate was added and the mixture was extracted with ethyl acetate. The extract was washed with brine, dried ($MgSO_4$) and evaporated to give an oil which was crystallised from methyl acetate-light petroleum (b.p. 60—80°) to give the *title compound* (530 mg) as white microcrystals, m.p. 111—113°.

| | | | |
|---|---|---|---|
| Found: | C, 59.9: | H, 7.8: | N, 23.0; |
| $C_{18}H_{28}N_6O_2$ requires: | C, 60.0: | H, 7.8; | N, 23.3% |

Example 16

*N*-Cyano-N′-[2-hydroxy-3-[3-(1-piperidinylmethyl)phenoxy]propyl]-N″-methylguanidine

A mixture of 1-amino-3[1-piperidinylmethyl)phenoxy)-2-propanol (1 g) and methyl N-cyano-N′-methylcarbamimidothioate (0.49 g) was heated at 100° under water pump vacuum for 4 h. The resulting brown glass was chromatographed on silica using dichloromethane:ethanol:ammonia (100:8:1) to give a brown gum (0.75 g). This gum was further purified by chromatography using methanol:ammonia (250:1) to give the *title compound* (0.27 g) as a white solid foam, m.p. 45—7° (softens).

n.m.r. ($CDCl_3$): 2.8, t, (1H); 3.03—3.37, m, (3H); 3.63, q, (1H); 3.91, t, (1H); 5.8—6.2 m, (3H); 6.47—6.79, m, (4H); 7.22, d, (3H); 7.68, m, (4H); 8.52, m, (6H).

Example 17

1-[(1-Methyl-1*H*-tetrazol-5-yl)amino]-4-[3-(1-piperidinylmethyl)phenoxy]-2-butanol

A solution of 1-amino-4-[3-(1-piperidinylmethyl)phenoxy]-2-butanol (1.39 g) and 5-bromo-1-methyl-1*H*-tetrazole (0.82 g) in absolute ethanol (10 ml) was heated in an autoclave at 125° for 18 h. The solution was evaporated and the residue dissolved in 1M hydrochloric acid (25 ml). The acidic solution was washed with ethyl acetate, basified with excess sodium carbonate and extracted with ethyl acetate. The extract was dried and evaporated to leave a solid which was triturated with dry diethyl ether to give a white solid (1.0 g). This solid was recrystallised from methyl acetate-light petroleum (b.p. 60—80°) to give the *title compound* (0.6 g) as white crystals, m.p. 138—9°.

| | | | |
|---|---|---|---|
| Found: | C, 59.8: | H, 7.9: | N, 23.2; |
| $C_{18}H_{28}N_6O_2$ requires: | C, 60.0: | H, 7.8; | N, 23.3% |

Example 18

5-[[2-Hydroxy-4-[3-(1-piperidinylmethyl)phenoxy]-butyl]amino)-1-methyl-1*H*-1,2,4-triazole-3-methanol

A solution of 1-amino-4-[3-(1-piperidinylmethyl)phenoxy]-2-butanol (1.4 g) and methyl N-[2-(acetyloxy)acetyl]-1-methyl-2-(phenylmethylene)-hydrazine-carboximidothioate (1.61 g) in toluene (20 ml) was stirred at 25° for 3 h. The solution was treated with 5N hydrocholoric acid (10 ml) and stirred for 16 h. The phases were separated, the aqueous phase was washed with toluene, basified with excess 2N sodium hydroxide and ethyl acetate (50 ml) was added. The liquid phases were decanted off from the resulting white solid which was recrystallised from ethyl acetate-methanol to give the *title compound* (193 mg) as white crystals, m.p. 147.5—148°.

| | | | |
|---|---|---|---|
| Found: | C, 61.6: | H, 8.1: | N, 17.8; |
| $C_{20}H_{31}N_5O_3$ requires: | C, 61.7: | H, 8.0; | N, 18.0% |

Examples of Pharmaceutical Compositions

| 1. Tablets | mg/tablets |
|---|---|
| Active ingredient | 5.0 to 25.0 |
| Lactose | 131.5 to 111.5 |
| Pregelatinised maize starch | 7.5 |
| Sodium starch glycollate | 4.5 |
| Magnesium stearate | 1.5 |
| Compression weight | 150.0 |

The active ingredient is sieved through a 250 µm sieve and blended with the lactose and pregelatinised maize starch. This mix is granulated by the addition of water. The granules are dried, screened and blended with the sodium starch glycollate and magnesium stearate. The lubricated granules are compressed into tablets using 8.0 mm diameter punches.

| 2. Injections | % w/w |
|---|---|
| Active ingredients | 0.5 |
| Water for injection B.P. to | 100.0 |

Sodium chloride may be added to adjust the toxicity of the solution and the pH may be adjusted to that of maximum stability using dilute acid or alkali or by the addition of suitable buffer salts.

The solution is prepared, clarified and filled into appropriate sized ampoules sealed by fusion of the glass. The injection is sterilized by heating in an autoclave using one of the acceptable cycles. Alternatively the solution may be sterilized by filtration and filled in sterile ampoules under aseptic conditions.

The solution may be packed under an inert atmosphere of nitrogen or other suitable gas.

The ability of the compounds to inhibit histamine induced gastric acid secretion was demonstrated in the perfused rat stomach test referred to above. For example the compounds of Examples 1 to 9 (a) have $ED_{50}$ values within the range of 0.05—0.74 mg/kg in the perfused rat stomach test.

The compounds of the invention have in general low toxicity at doses at which they effectively inhibit gastric acid secretion. Thus for example the compound of Example 4 produced no toxic effects when administered intravenously to anaesthetised rats at a dose at least 4 times its $ED_{50}$ value.

**Claims**

1. Compounds of the general formula (I)

$$R_1R_2NAlkQO(CH_2)_nCH(OR_5)(CH_2)_mNH{-}Z \quad (I)$$

and physiologically acceptable salts and hydrates thereof, in which

$R_1$ represents hydrogen, $C_{1-14}$ alkyl, $C_{3-8}$ cycloalkyl, $C_{3-6}$ alkenyl, $C_{3-6}$ alkynyl, aryl $C_{1-6}$ alkyl, trifluoro $C_{1-6}$ alkyl, heteroaralkyl or $C_{1-6}$ alkyl substituted by $C_{3-8}$ cycloalkyl, hydroxy, $C_{1-6}$ alkoxy, amino, $C_{1-6}$ alkylamino or di $C_{1-6}$ alkylamino; and

$R_2$ represents hydrogen or $C_{1-4}$ alkyl group; or

$R_1$ and $R_2$ together with the nitrogen atom to which they are attached form a 5 to 10 membered ring which may be saturated or may contain at least one double bond, may be unsubstituted or may be substituted by one or more $C_{1-3}$ alkyl groups or a hydroxy group and/or may contain another heteroatom selected from oxygen or sulphur;

Alk represents a straight or branched alkylene chain of 1 to 3 carbon atoms;

Q represents a benzene ring in which incorporation into the rest of the molecule is through bonds at the 1- and 3- or 1- and 4-positions;

$R_5$ represents hydrogen or acyl;

n and m, which may be the same or different, are each 1 or 2;

Z represents one of the groups;

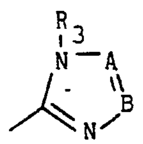 or 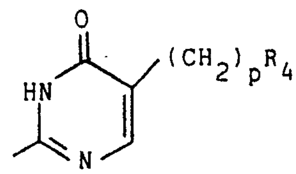 or 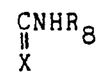

where

X represents NCN, $NSO_2$Methyl, $NSO_2$Phenyl or $CHNO_2$;

$R_8$ represents $C_{1-6}$ alkyl;

A represents N and B represents $CR_7$; or

A represents $CR_7$ and B represents N; or

A and B both represent N;

$R_3$ represents hydrogen, $C_{1-6}$ alkyl, $C_{3-6}$ alkenyl, aryl $C_{1-6}$ alkyl, hydroxy $C_{2-6}$ alkyl, $C_{1-6}$ alkoxy $C_{2-6}$ alkyl or $C_{1-4}$ alkanoyloxy $C_{2-6}$ alkyl;

$R_7$ represents hydrogen, $C_{1-6}$ alkyl, $C_{3-6}$ alkenyl, aryl $C_{1-6}$ alkyl, acyloxy $C_{1-6}$ alkyl, $C_{1-6}$ alkylthio $C_{1-6}$ alkyl, arylthio $C_{1-6}$ alkyl, aryloxy $C_{1-6}$ alkyl, aryl $C_{1-6}$ alkyloxy $C_{1-6}$ alkyl, or the group $(CH_2)_qR_6$ where q is zero, 1, 2, 3, 4, 5 or 6 and the alkylene chain $(CH_2)_q$ may be straight or branched, and

$R_6$ is hydroxy, $C_{1-6}$ alkoxy, nitro, heteroaryl, tetrahydropyranyloxy, or $CH_2NHC(=X)NHR_9$ where X is as defined above and $R_9$ is $C_{1-6}$ alkyl;

or $R_6$ is the group $NR_{10}R_{11}$, where $R_{10}$ is hydrogen or $C_{1-6}$ alkyl; and $R_{11}$ is hydrogen, $C_{1-6}$ alkyl, $C_{3-6}$ alkenyl, aryl, aryl $C_{1-6}$ alkyl, or heteroaralkyl, or $R_{11}$ is the group $SO_2R_{12}$ where $R_{12}$ is $C_{1-6}$ alkyl or aryl; or $R_{11}$ is the group $COR_{13}$ where $R_{13}$ is hydrogen, $C_{1-6}$ alkyl, aryl, aryl $C_{1-6}$ alkyl, $C_{1-6}$ alkoxy, halomethyl, heteroaryl, heteroaralkyl or the group $NHR_{14}$ where $R_{14}$ is hydrogen, $C_{1-6}$ alkyl, $C_{3-8}$ cycloalkyl, aryl or aryl $C_{1-6}$ alkyl; or $R_{10}$ and $R_{11}$ together represent the group $=CR_{15}R_{16}$ where $R_{15}$ represents aryl or heteroaryl and $R_{16}$ represents hydrogen or $C_{1-6}$ alkyl; or

$R_6$ is the group $SO_2R_{17}$ in which $R_{17}$ is hydroxy, $C_{1-6}$ alkyl, aryl or the group $NR_{18}R_{19}$ where $R_{18}$ and $R_{19}$, which may be the same or different, each represent hydrogen or $C_{1-6}$ alkyl; or

$R_6$ is the group $COR_{20}$ where $R_{20}$ is hydrogen, hydroxy, $C_{1-6}$ alkoxy, aryloxy, aryl $C_{1-6}$ alkyloxy, $C_{1-6}$ alkyl, aryl, aryl $C_{1-6}$ alkyl, or the group $NR_{21}R_{22}$ where $R_{21}$ is hydrogen or $C_{1-6}$ optionally substituted by a hydroxy or $C_{1-6}$ alkoxy group; and $R_{22}$ is hydrogen, $C_{1-6}$ alkyl (optionally substituted by a hydroxy or $C_{1-6}$ alkoxy group), $C_{3-6}$ alkenyl, aryl, aryl $C_{1-6}$ alkyl or $C_{3-8}$ cycloalkyl, or $NR_{21}R_{22}$ forms a 5 to 8 membered ring which may contain another heteroatom, e.g. oxygen, or a double bond and/or may be substituted by hydroxy or one or two $C_{1-3}$ alkyl groups; or $R_6$ is the group $CR_{23}=NR_{24}$ where $R_{23}$ is hydrogen, $C_{1-6}$ alkyl, aryl or aryl $C_{1-6}$ alkyl and $R_{24}$ is hydroxy, $C_{1-6}$ alkoxy, aryl $C_{1-6}$ alkyloxy or $-NHC(=Y)NH_2$ where Y is oxygen or sulphur;

or when A represents $CR_7$ and B represents N, the groups $R_3$ and $R_7$ taken together represent $-(CH=CH)_2-$ or $-(CH_2)_4-$;

with the proviso (1) that when the group $R_6$ contains a carbon atom through which it is linked to the alkylene group $(CH_2)_q$ then the total number of carbon atoms in the resulting chain is not greater than 6 (i.e. q is not greater than 5);

$R_4$ represents phenyl, phenoxy or pyridinyl which may optionally be substituted by one or more halogen, $C_{1-6}$ alkyl, $C_{1-6}$ alkoxy $C_{1-6}$ alkyl or $C_{1-6}$ alkoxy groups or by a methylenedioxy group;

p represents an integer which is 1, 2 or 3;

with the following further provisos;

(2) the term aryl within the definitions of $R_1$, $R_3$, $R_6$ and $R_7$ means phenyl or phenyl substituted by one or more $C_{1-3}$ alkyl groups or $C_{1-6}$ alkoxy groups or halogen atoms;

(3) the term heteroaryl as a group or part of a group within the definitions of $R_1$ and $R_6$ means a 5 or 6 membered monocyclic ring containing from 1 to 3 heteroatoms selected from oxygen, nitrogen and sulphur, which may be unsubstituted or substituted by $C_{1-3}$ alkyl, $C_{1-3}$ alkoxy, hydroxy $C_{1-6}$ alkyl, amino $C_{1-6}$ alkyl, $C_{1-6}$ alkylamine $C_{1-6}$ alkyl, di $C_{1-6}$ alkylamino $C_{1-6}$ alkyl or halogen;

(4) the alkyl portion of a heteroaralkyl group within the definitions of $R_1$ and $R_6$ is a straight or branched $C_{1-4}$ alkyl chain and the heteroaryl ring is linked to the alkyl portion through a carbon atom;

(5) the term acyl as a group or part of a group within the definitions of $R_5$ and $R_7$ means an aroyl or aryl $C_{2-7}$ alkanoyl group (in which the term aryl is as defined as in proviso 2) or a $C_{1-6}$ alkanoyl group.

(6) the following compounds are excluded:

(i) N-[3-[3-piperidinylmethyl)phenoxy]-2-hydroxypropyl]-N′-methyl-N″-cyanoguanidine;

(ii) N-[3-[3-(dimethylaminomethyl)phenoxy]-2-acetoxypropyl]-N′-methyl-N″-cyanoguanidine;

(iii) 1-[N-3-[3-(dimethylaminomethyl)phenoxy]-2-hydroxypropylamino]-1-N-methylamino-2-nitroethene;

(iv) 1-[N-3-[3-(1′-cyclopropylmethylaminomethyl)phenoxy]-2-hydroxy-propylamino]-1-N-methylamino-2-nitroethene;

(v) 2-N-[3-[3-(dimethylaminomethyl)phenoxy]-2-hydroxypropyl]amino-5-[(6-methyl-3-pyridyl)methyl]-pyrimidinin-4(3H)-one;

(vi) N-3-[3-dimethylaminomethyl)phenoxy-2-hydropropyl]-N′-methyl-N″-cyanoguanidine.

2. Compounds as claimed in claim 1 in which

$R_1$ represents $C_{1-8}$ alkyl, $C_{1-4}$ alkyl substituted by a trifluoromethyl group, $C_{2-4}$ alkyl substituted by hydroxy or a di $C_{1-3}$ alkyl amino group, $C_{5-7}$ cycloalkyl, $C_{3-5}$ alkenyl, phenyl $C_{1-3}$ alkyl, or a heteroaryl $C_{1-3}$ alkyl group where the heteroaryl ring contains one heteroatom;

$R_2$ represents hydrogen or methyl; or

$R_1R_2N$ represents a 5 to 7 membered ring optionally containing a double bond, an oxygen atom or a $C_{1-6}$ alkyl substituent;

Alk represents methylene;

$R_3$ represents hydrogen, or $C_{1-6}$ alkyl, or hydroxy $C_{2-4}$ alkyl or when A is the group $CR_7$ then $R_3$ and $R_7$, taken together, represent $-(CH=CH)_2-$ or $-(CH_2)_4-$;

Q represents a benzene ring incorporated into the rest of the molecule through bonds at the 1- and 3-positions;

$R_5$ represents hydrogen or $C_{1-6}$ alkanoyl;

n and m both represent 1, or one of n and m represents 2;

$R_7$ represents $C_{1-6}$ alkoxy $C_{1-6}$ alkyl, $C_{1-6}$ alkylthio $C_{1-6}$ alkyl, $C_{1-6}$ alkanoyloxy $C_{1-6}$ alkyl, benzene, or $-CH=NOH$; or

$R_7$ represents the group $(CH_2)_qR_6$ where q is zero, 1, 2, 3, and $R_6$ represents hydroxyl, $CH_2NHSO_2R_{12}$ (where $R_{12}$ is $C_{1-6}$ alkyl), $CH_2NHC(=X)NHCH_3$ (where X=NCN or $CHNO_2$), $SO_2R_{17}$ (where $R_{17}$ is $C_{1-6}$ alkyl), or $R_6$ represents $COR_{20}$ where $R_{20}$ is hydroxyl or $NR_{21}R_{22}$ and $R_{21}$ and $R_{22}$ independently represent hydrogen or $C_{1-3}$ alkyl or $R_{21}$ and $R_{22}$ together with the nitrogen atom to which they are attached represent a pyrrolidino ring, or

$R_6$ represents the group $NR_{10}R_{11}$ where $R_{10}$ represents hydrogen and $R_{11}$ is hydrogen or $COR_{13}$, where $R_{13}$ represents hydrogen, $C_{1-6}$ alkyl, phenyl, benzyl, $C_{1-6}$ alkoxy, NH phenyl, or $R_{10}$ and $R_{11}$ together represent the group $=CHR_{15}$ where $R_{15}$ is phenyl or pyridyl;

$R_4$ represents pyridinyl optionally substituted by a $C_{1-6}$ alkyl group; or a phenyl group optionally substituted by a $C_{1-6}$ alkoxy group;

p represents 1;

$R_8$ represents $C_{1-6}$ alkyl;

X represents $CHNO_2$.

3. Compounds as claimed in claim 1 of the general formula (II)

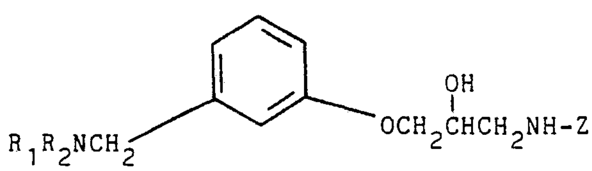

(II)

in which $R_1R_2N$ is dimethylamino, pyrrolidino, piperidino or hexamethylenimino; and Z represents

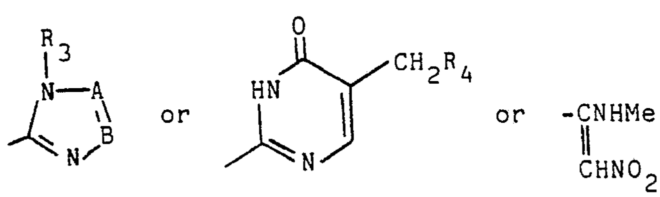

where, when B represents $CR_7$, then $R_3$ is hydrogen or methyl and $R_7$ is hydroxymethyl, amino, $C_{1-6}$ alkanoyloxy $C_{1-6}$ alkyl N=CHPh, $(CH_2)_3$ NHC(=$CHNO_2$)NHMe, aminopropyl, methylsulphonylmethyl or acetylamino; or when A is $CR_7$ then $R_3$ is methyl and $R_7$ is amino or $R_3$ and $R_7$ together form the group $-(CH=CH)_2-$or $(CH_2)_4$; or when A and B both represent N then $R_3$ is methyl; and $R_4$ is 3-pyridinyl, 6-methyl-3-pyridinyl or 4-methoxyphenyl.

4. 1-[3-Amino-1-methyl-1H-1,2,4-triazol-5-yl)amino]-3-[3-(1-piperidinylmethyl)phenoxy]-2-propanol;

5-[[2-hydroxy-3-[3-(1-piperidinylmethyl)phenoxy]propyl]amino]-1-methyl-1*H*-1,2,4-triazole-3-methanol;

1-[(1-methyl-1H-tetrazol-5-yl)amino]-3-[3-(1-piperidinylmethyl)-phenoxy]-2-propanol;

2-[[2-hydroxy-3-[3-(1-piperidinylmethyl]phenoxy]propyl]amino]-5-[(6-methyl-3-pyridinyl)methyl]-4-(3*H*)-pyrimidinone;

2-[[2-hydroxy-3-[3-(1-piperidinylmethyl)phenoxy]propyl]amino]-5-(3-pyridinylmethyl)-4(3H)-pyrimidinone;

2-[[2-hydroxy-3-[3-(1-piperidinylmethyl)phenoxy]propyl]amino]-5-[(4-methoxyphenyl)methyl]-4-(3H)-pyrimidinone; and physiologically acceptable salts thereof.

5. A process for the preparation of compounds as claimed in claim 1 which comprises;

a) for the preparation of compounds of formula (I) in which $R_5$ represents hydrogen and Z represents the group

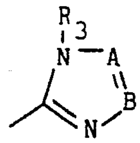

where either A represents $CR_7$ and B represents N or A represents N and B represents $CR_7$, and $R_7$ is other than acyloxyalkyl, alkoxy, nitro, $(CH_2)_qN{=}CR_{15}R_{16}$, $SO_2R_{17}$, $COR_{20}$ (where $R_{20}$ is hydrogen, aryl or aralkyl) or $CR_{23}{=}NR_{24}$, or when A represents $CR_7$ and B represents N then $R_3$ and $R_7$ may not represent the group $-(CH{=}CH)_2-$ or $-(CH_2)_4-$, cyclising a compound of formula (III)

$$\begin{array}{c} \qquad\qquad\qquad OR_5 \qquad\qquad\qquad\qquad R_{25} \\ R_1R_2NAlkQO(CH_2)_nCH(CH_2)_mNHC{-}N{-}NHY' \\ \qquad\qquad\qquad\qquad\qquad\quad \| \\ \qquad\qquad\qquad\qquad\qquad\quad V \end{array} \qquad (III)$$

in which $R_{25}$ is as defined in claim 1 for $R_3$, V' is

$$\begin{array}{c} NCR_7' \\ \| \\ V \end{array}$$

or NCN and Y' is hydrogen where V is oxygen or sulphur and $R_7'$ is a group as defined in claim 1 for $R_7$ or a group convertible thereto under the conditions of the cyclisation reaction or $R_7'$ represents halogen or alkoxy; or V' is $NR_3$, $R_{25}$ is hydrogen and Y' is

$$\begin{array}{c} CR_7 \\ \| \\ Y'' \end{array}$$

where Y'' is sulphur, oxygen or NH;

b) for the preparation of compounds of formula (I) in which $R_5$ is hydrogen and Z is as defined in claim 1 except $C(=X)NHR_8$ and except that when A or B represent the group $CR_7$ then $R_7$ represents nitro or when A represents $CR_7$ then $R_3$ and $R_7$ together represent the group $-(CH{=}CH)_2-$, heating the diamine (VII)

$$\begin{array}{c} \qquad\qquad\qquad OR_5 \\ \qquad\qquad\qquad | \\ R_1R_2NAlkQO(CH_2)_nCH(CH_2)_mNH_2 \end{array} \qquad (VII)$$

in which $R_5$ represents hydrogen with a compound of formula (I) or (X)

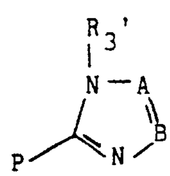

(IX)

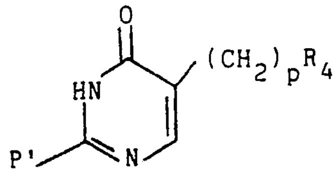

(X)

in which $R_3'$ is the group $R_3$ or a group convertible thereto, and P and P' are leaving groups;

c) for the production of compounds of formula (I) in which Z represents

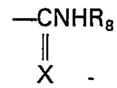

and $R_5$ represents hydrogen, reacting an amine of the formula (XI)

$$R_{26}NH_2 \qquad (XI)$$

with a compound of general formula (XII)

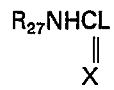

(XII)

where one of the groups $R_{26}$ and $R_{27}$ represents the group

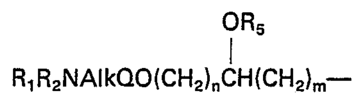

and the other represents the group $R_8$, and L is a leaving group;

d) for the production of compounds of formula (I) in which $R_5$ represents hydrogen and A represents N and B represents $CR_7$ or A represents $CR_7$ and B represents N, where $R_7$ is the group $(CH_2)_qR_6$ where $R_6$ is $NR_{10}COR_{13}$, $NR_{10}SO_2R_{12}$, $CH_2NHC(=X)NHR_8$ or $N=CR_{15}R_{16}$ treating an aminoalkyltriazole of formula (XIV)

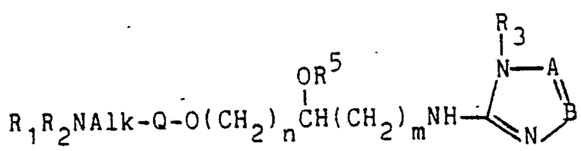

(XIV)

in which $R_1$, $R_2$ and $R_3$ are as defined in claim 1 or are groups readily convertible thereto, A represents N and B represents $CR_{28}$ or A represents $CR_{28}$ and B represents N, where $R_{28}$ is the group $(CH_2)_qNHR_{10}$ the group $(CH_2)_{q+1}NH_2$ or the group $(CH_2)_qNH_2$, with a compound capable of replacing the hydrogen atom in the group $NHR_{10}$ by the group $COR_{13}$ or $SO_2R_{12}$ or a hydrogen atom in the group $NH_2$ of the group $(CH_2)_{q+1}NH_2$ by the group $C(=X)NHR_9$ or both hydrogen atoms in the group $NH_2$ of the group $(CH_2)_qNH_2$ by the group $=CR_{15}R_{16}$;

e) for the production of compounds of formula (I) in which $R_7$ is an acyloxyalkyl group and/or $R_5$ is acyl, treating the corresponding compound of formula (I) in which $R_7$ is hydroxyalkyl group and/or $R_5$ is hydrogen with either an appropriate acid or an activated derivative thereof;

f) for the production of compounds of formula (I) in which A is $CR_7$ and B is N, $R_3$ and $R_7$ together represent $-(CH_2)_4-$ and $R_5$ is hydrogen, reducing the corresponding compound in which $R_3$ and $R_7$ together represent $-(CH=CH)_2-$;

g) for the production of compounds of formula (I) in which Z is

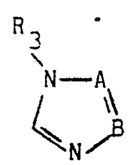

Alk is $CH_2$ and $R_5$ is hydrogen treating an aldehyde of formula (XV)

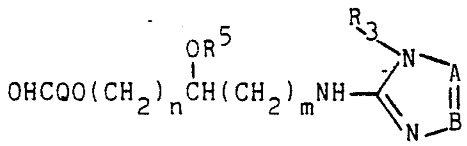

(XV)

with an amine $R_1R_2NH$, followed by reduction;

h) for the production of compounds of formula (I) in which $R_5$ is hydrogen and $R_6$ is tetrahydropyranyloxy, reacting the corresponding hydroxyalkyltriazole of formula (I) with dihydropyran;

i) for the production of a compound of formula (I) in the form of a physiologically acceptable salt, converting the compound of formula (I) in the form of the free base into a physiologically acceptablke salt.

6. A pharmaceutical composition which comprises a compound as claimed in any of claims 1 to 4 together with at least one inert pharmaceutically acceptable carrier or diluent.

7. Compounds of the general formula (VII)

$$R_1R_2NAlkQO(CH_2)_nCH(OR^5)(CH_2)_mNH_2 \qquad (VII)$$

in which $R_1$, $R_2$, Alk, Q, $R_5$, n and m are as defined in claim 1;

the following compound being excluded:

3-(3-amino-2-hydroxypropoxy)-N,N-dimethylbenzlamine.

8. Compounds as claimed in claim 7 in which $R_1$, $R_2$, Alk, Q, $R_5$, n an m are as defined in claim 2.

9. Compounds as claimed in claim 7 in which $R_1$, $R_2$, Alk, Q, $R_5$, n and m are as defined in claim 3.

**Patentansprüche**

1. Verbindungen der allgemeinen Formel (I)

$$R_1R_2NAlkQO(CH_2)_nCH(OR_5)(CH_2)_mNH{-}Z \qquad (I)$$

und die physiologisch annehmbaren Salze und Hydrate davon, worin

$R_1$ für Wasserstoff, $C_{1-14}$-Alkyl, $C_{3-8}$-Cycloalkl, $C_{3-6}$-Alkenyl, $C_{3-6}$-Alkinyl, Aryl-$C_{1-6}$-alkyl, Trifluor-$C_{1-6}$-alkyl, Heteroaralkyl oder $C_{1-6}$-Alkyl, substituiert durch $C_{3-8}$-Cycloalkyl, Hydroxy, $C_{1-6}$-Alkoxy, Amino, $C_{1-6}$-Alkylamino oder di-$C_{1-6}$-alkylamino steht, und

$R_2$ für Wasserstoff oder eine $C_{1-4}$-Alkylgruppe steht, oder

$R_1$ und $R_2$ zusammen mit dem Stickstoffatom, an das sie angefügt sind, einen 5- bis 10gliedrigen Ring bilden, der gesättigt sein kann oder der mindestens eine Doppelbindung enthalten kann, der unsubstituiert sein kann oder der durch ein oder mehrere $C_{1-3}$-Alkylgruppen oder eine Hydroxygruppen substituiert sein kann und/oder der ein weiteres Heteroatom, ausgewählt aus Sauerstoff oder Schwefel, enthalten kann,

Alk für eine geradkettige oder verzweigte Alkylenkette mit 1 bis 3 Kohlenstoffatomen steht,

Q für einen Benzolring, dessen Einarbeitung in den Rest des Moleküls durch Bindungen in 1- und 3- oder 1- und 4-Stellungen erfolgt ist,

$R_5$ für Wasserstoff oder Acyl steht,

n und m, die gleich oder verschieden sein können, jeweils 1 oder 2 sind,

Z für eine der Gruppen

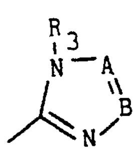 oder 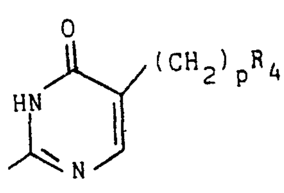 oder 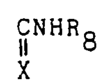

steht, worin X für NCN, $NSO_2$Methyl, $NSO_2$Phenyl oder $CHNO_2$ steht,

$R_8$ für $C_{1-6}$-Alkyl steht,

A für N und B für $CR_7$ steht, oder

A für $CR_7$ und B für N steht, oder

A und B beide für N stehen,

$R_3$ für Wasserstoff, $C_{1-6}$-Alkyl, $C_{3-6}$-Alkenyl, Aryl-$C_{1-6}$-alkyl, Hydroxy-$C_{2-6}$-alkyl, $C_{1-6}$-Alkoxy-$C_{2-6}$-alkyl oder $C_{1-4}$-Alkanoyloxy-$C_{2-6}$-alkyl steht,

$R_7$ für Wasserstoff, $C_{1-6}$-Alkyl, $C_{3-6}$-Alkenyl, Aryl-$C_{1-6}$-alkyl, Acyloxy-$C_{1-6}$-alkyl, $C_{1-6}$-Alkylthio-$C_{1-6}$-alkyl, Arylthio-$C_{1-6}$-alkyl, Aryloxy-$C_{1-6}$-alkyl, Aryl-$C_{1-6}$-alkyloxy-$C_{1-6}$-alkyl oder die Gruppe $(CH_2)_qR_6$, worin q den Wert Null, 1, 2, 3, 4, 5 oder 6 hat, und die Alkylenkette $(CH_2)_q$ geradkettig oder verzweigt sein kann, und

$R_6$ für Hydroxy, $C_{1-6}$-Alkoxy, Nitro, Heteroaryl, Tetrahydropyranyloxy oder $CH_2NHC(=X)NHR_9$ steht, worin X wie oben definiert ist und $R_9$ für $C_{1-6}$-Alkyl steht, oder

$R_6$ die Gruppe $NR_{10}R_{11}$ ist, worin $R_{10}$ für Wasserstoff oder $C_{1-6}$-Alkyl steht, und $R_{11}$ für Wasserstoff, $C_{1-6}$-Alkyl, $C_{3-6}$-Alkenyl, Aryl, Aryl-$C_{1-6}$-alkyl oder Heteroaralkyl steht, oder $R_{11}$ für die Gruppe $SO_2R_{12}$ steht, worin $R_{12}$ für $C_{1-6}$-Alkyl oder Aryl steht, oder $R_{11}$ für die Gruppe $COR_{13}$ steht, worin $R_{13}$ für Wasserstoff, $C_{1-6}$-Alkyl, Aryl, Aryl-$C_{1-6}$-alkyl, $C_{1-6}$-Alkoxy, Halomethyl, Heteroaryl, Heteroaralkyl oder die Gruppe $NHR_{14}$ steht, worin $R_{14}$ für Wasserstoff, $C_{1-6}$-Alkyl, $C_{3-8}$-Cycloalkyl, Aryl oder Aryl-$C_{1-6}$-alkyl steht, oder $R_{10}$ und $R_{11}$ zusammen die Gruppe $=CR_{15}R_{16}$ darstellen, worin $R_{15}$ für Aryl oder Heteroaryl steht, und $R_{16}$ für Wasserstoff oder $C_{1-6}$-Alkyl steht, oder

$R_6$ die Gruppe $SO_2R_{17}$ ist, worin $R_{17}$ für Hydroxy, $C_{1-6}$-Alkyl, Aryl oder die Gruppe $NR_{18}R_{19}$ steht, worin $R_{18}$ und $R_{19}$, die gleich oder verschieden sein können, jeweils für Wasserstoff oder $C_{1-6}$-Alkyl stehen, oder

$R_6$ die Gruppe $COR_{20}$ ist, worin $R_{20}$ für Wasserstoff, Hydroxy, $C_{1-6}$-Alkoxy, Aryloxy, Aryl-$C_{1-6}$-alkyloxy, $C_{1-6}$-Alkyl, Aryl, Aryl-$C_{1-6}$-alkyl, oder die Gruppe $NR_{21}R_{22}$ steht, worin $R_{21}$ für Wasserstoff oder $C_{1-6}$, das gegebenenfalls durch eine Hydroxy- oder $C_{1-6}$-Alkoxygruppe substituiert ist, steht, und $R_{22}$ für Wasserstoff, $C_{1-6}$-Alkyl (das gegebenenfalls durch eine Hydroxy- oder $C_{1-6}$-Alkoxygruppe substituiert ist), $C_{3-6}$ Alkenyl, Aryl, Aryl-$C_{1-6}$-Alkyl oder $C_{3-8}$Cycloalkyl steht, oder $NR_{21}R_{22}$ einen 5- bis 8gliedrigen Ring bildet, der ein weiteres Heteroatom, z.B. Sauerstoff oder eine Doppelbindung enthalten kann und/oder durch eine Hydroxy- oder ein oder zwei $C_{1-3}$-Alkylgruppen substituiert sein kann, oder $R_6$ für die Gruppe $CR_{23}=NR_{24}$ steht, worin $R_{23}$ für Wasserstoff, $C_{1-6}$-Alkyl, Aryl oder Aryl-$C_{1-6}$-alkyl steht, und $R_{24}$ für Hydroxy, $C_{1-6}$-Alkoxy, Aryl-$C_{1-6}$-alkyloxy oder $NHC(=Y)NH_2$ steht, worin Y für Sauerstoff oder Schwefel steht,

oder wenn A für $CR_7$ und B für N steht, die Gruppen $R_3$ und $R_7$ zusammengenommen $\text{-(-CH=CH-)}_2\text{-}$ oder $-(CH_2)_4-$ darstellen,

mit der Maßgabe (1), daß, wenn die Gruppe $R_6$ ein Kohlenstoffatom enthält, durch das sie mit der Alkylengruppe $(CH_2)_q$ verbunden ist, dann die Gesamtanzahl von Kohlenstoffatomen in der resultierenden Kette nicht größer als 6 ist (d.h. q nicht größer als 5 ist),

$R_4$ für Phenyl, Phenoxy oder Pyridinyl steht, das gegebenenfalls durch ein oder mehrere Halogen-, $C_{1-6}$-Alkyl-, $C_{1-6}$-Alkoxy-$C_{1-6}$-alkyl- oder $C_{1-6}$-Alkoxygruppen oder durch eine Methylendioxygruppe substituiert sein kann,

p für eine ganze Zahl steht, die 1, 2 oder 3 ist,

mit den folgenden weiteren Maßgaben:

(2) die Bezeichnung Aryl innerhalb der Definitionen von $R_1$, $R_3$, $R_6$ und $R_7$ bedeutet Phenyl oder Phenyl, das durch ein oder mehrere $C_{1-3}$-Alkylgruppen oder $C_{1-6}$-Alkoxygruppen oder Halogenatome substituiert ist,

(3) die Bezeichnung Heteroaryl als Gruppe oder Teil einer Gruppe innerhalb der Definitionen von $R_1$ und $R_6$ bedeutet einen 5- oder 6gliedrigen monocyclischen Ring, enthaltend 1 bis 3 Heteroatome, ausgewählt aus Sauerstoff, Stickstoff und Schwefel, der unsubstituiert sein kann oder durch $C_{1-3}$-Alkyl, $C_{1-3}$-Alkoxy, Hydroxy-$C_{1-6}$-alkyl, Amino-$C_{1-6}$-alkyl, $C_{1-6}$-Alkylamino-$C_{1-6}$-alkyl, di-$C_{1-6}$-alkylamino-$C_{1-6}$-alkyl oder Halogen substituiert sein kann,

(4) der Alkylteil einer Heteroarylgruppe innerhalb der Definitionen von $R_1$ und $R_6$ ist eine geradkettige oder verzweigte $C_{1-4}$-Alkylkette, und der Heteroarylring ist mit dem Alkylteil durch ein Kohlenstoffatom verbunden,

(5) die Bezeichnung Acyl als Gruppe oder Teil einer Gruppe innerhalb der Definitionen von $R_5$ und $R_7$ bedeutet eine Aroyl- oder Aryl-$C_{2-7}$-Alkanoylgruppe (worin die Bezeichung Aryl wie im Zusammenhang mit der Maßgabe (2) definiert ist) oder eine $C_{1-6}$-Alkanoylgruppe,

(6) die folgenden Verbindung sind ausgenommen:

(i) N-[3-[3-Piperidinylmethyl)phenoxy]-2-hydroxypropyl]-N'-methyl-N''-cyanoguanidin;

(ii) N-[3-[3-(Dimethylaminomethyl)phenoxy]-2-acetoxypropyl]-N'-methyl-N''-cyanoguanidin;

(iii) 1-[N-3-[3-(Dimethylaminomethyl)phenoxy]-2-hydroxy-propylamino]-1-N-methylamino-2-nitroethen;

(iv) 1-[N-3-[3-(1'-Cyclopropylmethylaminomethyl)phenoxy]-2-hydroxy-propylamino]-1-N-methylamino-2-nitroethen;

(v) 2-N-[3-[3-(Dimethylaminomethyl)phenoxy]-2-hydroxypropyl]amino-5-[(6-methyl-3-pyridyl)methyl]-pyrimidinin-4-(3H)-on;

(vi) N-3-[3-Dimethylaminomethyl)phenoxy-2-hydropropyl]-N'-methyl-N''-cyanoguanidin.

2. Verbindung nach Anspruch 1, dadurch gekennzeichnet, daß

$R_1$ für $C_{1-8}$-Alkyl, $C_{1-4}$-Alkyl, das durch eine Trifluormethylgruppe substituiert ist, $C_{2-4}$-Alkyl, das durch eine Hydroxy- oder eine di-$C_{1-3}$-Alkylaminogruppe substituiert ist, $C_{5-7}$-Cycloalkyl-, $C_{3-5}$-Alkenyl-, Phenyl-$C_{1-3}$-alkyl- oder eine Heteroaryl-$C_{1-3}$-alkylgruppe, wobei der Heteroarylring ein Heteroatom enthält, steht,

$R_2$ für Wasserstoff oder Methyl steht, oder

$R_1R_2$,N für einen 5- bis 7gliedrigen Ring steht, der gegebenenfalls eine Doppelbindung, ein Sauerstoffatom oder einen $C_{1-6}$-Alkylsubstituenten enthält,

Alk für Methylen steht,

$R_3$ für Wasserstoff oder $C_{1-6}$-oder Hydroxy-$C_{2-4}$-alkyl steht, oder wenn A die Gruppe $CR_7$ ist, dann $R_3$ und $R_7$ miteinander genommen $\{-CH=CH\}_2$ oder $-(CH_2)_4-$ darstellen,

Q für einen Benzolring steht, der in den Rest des Moleküls durch Bindungen in 1- und 3-Stellungen eingearbeitet ist,

$R_5$ für Wasserstoff oder $C_{1-6}$-Alkanoyl steht,

n und m beide den Wert 1 haben oder eines von n und m den Wert 2 hat,

$R_7$ für $C_{1-6}$-Alkoxy-$C_{1-6}$-alkyl, $C_{1-6}$-Alkylthio-$C_{1-6}$-alkyl, $C_{1-6}$-Alkanoyloxy-$C_{1-6}$-alkyl, Benzol oder $-CH=NOH$ steht, oder

$R_7$ für die Gruppe $(CH_2)_qR_6$ steht, worin q den Wert Null, 1, 2, 3 hat und $R_6$ für Hydroxyl, $CH_2NHSO_2R_{12}$ (worin $R_{12}$ für $C_{1-6}$-Alkyl steht), $CH_2NHC(=X)NHCH_3$ (worin X=NCN oder $CHNO_2$), $SO_2R_{17}$ (worin $R_{17}$ für $C_{1-6}$-Alkyl steht) steht, oder $R_6$ für $COR_{20}$ steht, worin $R_{20}$ für Hydroxyl oder $NR_{21}R_{22}$ steht, und $R_{21}$ und $R_{22}$ unabhängig voneinander für Wasserstoff oder $C_{1-3}$-Alkyl stehen, oder $R_{21}$ und $R_{22}$ zusammen mit dem Stickstoffatom, an das sie angefügt sind, einen Pyrrolidinoring darstellen, oder

$R_6$ für die Gruppe, $NR_{10}R_{11}$ steht, worin $R_{10}$ für Wasserstoff steht, und $R_{11}$ für Wasserstoff oder $COR_{13}$ steht, worin $R_{13}$ für Wasserstoff, $C_{1-6}$-Alkyl, Phenyl, Benzyl, $C_{1-6}$-Alkoxy, NH-Phenyl steht, oder $R_{10}$ und $R_{11}$ miteinander die Gruppe $=CHR_{15}$ darstellen, worin $R_{15}$ für Phenyl oder Pyridyl steht,

$R_4$ für Pyridinyl, das gegebenenfalls durch eine $C_{1-6}$-Alkylgruppe substituiert ist, oder eine Phenylgruppe, die gegebenenfalls durch eine $C_{1-6}$-Alkoxygruppe substituiert ist, steht,

p den Wert 1 hat,

$R_8$ für $C_{1-6}$-Alkyl steht,

X für $CHNO_2$ steht.

3. Verbindungen nach Anspruch 1 der allgemeinen Formel (II)

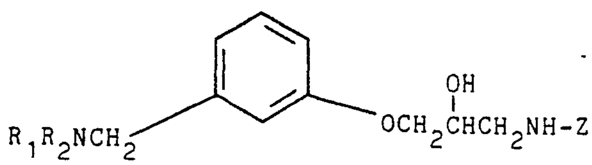

(II)

worin $R_1R_2N$ für Dimethylamino, Pyrrolidino, Piperidino oder Hexamethylenimino steht, und Z für

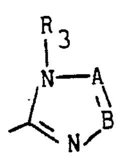

oder

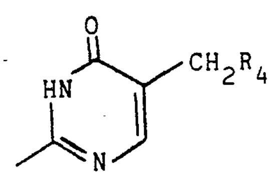

oder

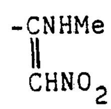

worin, wenn B für $CR_7$ steht, dann $R_3$ für Wasserstoff oder Methyl steht und $R_7$ Hydroxymethyl, Amino, $C_{1-6}$-Alkanoyloxy-$C_{1-6}$-alkyl, N=CHPh, $(CH_2)_3$ $NHC(=CHNO_2)NHMe$, Aminopropyl, Methylsulphonylmethyl oder Acetylamino ist, oder wenn A für $CR_7$ steht, dann $R_3$ Methyl ist und $R_7$ Amino ist, oder $R_3$ und $R_7$ miteinander die Gruppe $\{-CH=CH\}_2$ oder $(CH_2)_4$ bilden, oder wenn A und B beide für N stehen, dann $R_3$ Methyl ist, und $R_4$ 3-Pyridinyl, 6-Methyl-3-pyridinyl oder 4-Methoxyphenyl ist.

4. 1-[3-Amino-1-methyl-1H-1,2,4-triazol-5-yl)amino]-3-[3-(1-piperidinylmethyl)phenoxy]-2-propanol;

5-[[2-Hydroxy-3-[3-(1-piperidinylmethyl)phenoxy]propyl]amino]-1-methyl-1*H*-1,2,4-triazole-3-methanol;

1-[(1-Methyl-1H-tetrazol-5-yl)amino]-3-[3-(1-piperidinylmethyl)-phenoxy]-2-propanol;

2-[[-Hydroxy-3-[3-(1-piperidinylmethyl]phenoxy]propyl]amino]-5-[(6-methyl-3-pyridinyl)methyl]-4-(3*H*)-pyrimidinon;

2-[[2-Hydroxy-3-[3-(1-piperidinylmethyl)phenoxy]propyl]amino]-5-(3-pyridinylmethyl)-4-(3H)-pyrimidinon;

2-[[2-Hydroxy-3-[3-(1-piperidinylmethyl)phenoxy]propyl]amino]-5-[(4-methoxyphenyl)methyl]-4-(3H)-pyrimidinon; und die physiologisch annehmbaren Salze davon.

5. Verfahren zur Herstellung von Verbindungen nach Anspruch 1, dadurch gekennzeichnet, daß man a) zur Herstellung von Verbindungen der Formel (I), worin $R_5$ für Wasserstoff steht und Z für die Gruppe

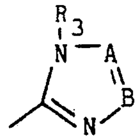

steht, worin entweder A für $CR_7$ steht und B für N steht, oder A für N steht und B für $CR_7$ steht, und $R_7$ eine andere Bedeutung als Acyloxyalkyl, Alkoxy, Nitro, $(CH_2)_qN{=}CR_{15}R_{16}$, $SO_2R_{17}$, $COR_{20}$ (worin $R_{20}$ für Wasserstoff, Aryl oder Aralkyl steht) oder $CR_{23}{=}NR_{24}$ hat, oder wenn A für $CR_7$ steht und B für steht, dann $R_3$ und $R_7$ nicht die Gruppe $\left(-CH{=}CH-\right)_2$ oder $-(CH_2)_4-$ darstellen, eine Verbindung der Formel (III)

$$R_1R_2NAlkQO(CH_2)_n\overset{\displaystyle OR_5}{\overset{|}{C}}H(CH_2)_mNH\underset{\underset{\displaystyle V}{\|}}{C}-\overset{\displaystyle R_{25}}{\overset{|}{N}}-NHY' \qquad (III)$$

worin $R_{25}$ wie in Anspruch 1 für $R_3$ definiert ist, V' für

$$\underset{\underset{\displaystyle V}{\|}}{NCR_7'}$$

oder NCN steht und V' für Wasserstoff steht, wobei V für Sauerstoff oder Schwefel steht und $R_7'$ eine Gruppe ist, wie sie in Anspruch 1 für $R_7$ definiert ist, oder eine Gruppe ist, die unter den Bedingungen der Cyclisierungsreaktion in eine solche Gruppe umwandelbar ist, oder $R_7'$ für halogen oder Alkoxy steht, oder V' für $NR_3$ steht, $R_{25}$ für Wasserstoff steht und Y' für

$$\underset{\underset{\displaystyle Y''}{\|}}{CR_7}$$

steht, wobei Y'' für Schwefel, Sauerstoff oder NH steht, cyclisiert,

(b) zur Herstellung von Verbindungen der Formel (I), worin $R_5$ für Wasserstoff steht und Z die in Anspruch 1 angegebenen Definition, ausgenommen $C({=}X)NHR_8$ und ausgenommen, daß, wenn A oder B die Gruppe $CR_7$ ist, dann $R_7$ für nitro steht, oder wenn A die Bedeutung $CR_7$ hat, dann $R_3$ und $R_7$ miteinander die Gruppe $\left(-CH{=}CH-\right)_2$ darstellen, hat, das Diamin (VII)

$$R_1R_2NAlkQO(CH_2)_n\overset{\displaystyle OR_5}{\overset{|}{C}}H(CH_2)_mNH_2 \qquad (VII)$$

worin $R_5$ für Wasserstoff steht, mit einer Verbindung der Formel (IX) oder (X)

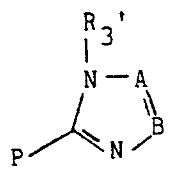

(IX)

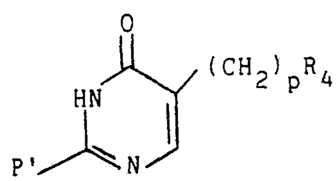

(X)

worin $R_3'$ für die Gruppe $R_3$ oder für eine in eine solche Gruppe umwandelbare Gruppe steht, und P und P' Austrittsgruppen sind, erhitzt,

(c) zur Herstellung von Verbindungen der Formel (I), worin Z für

$$-\underset{\underset{\displaystyle X}{\|}}{C}NHR_8$$

steht und $R_5$ für Wasserstoff steht, ein Amin der Formel (XI)

$$R_{26}NH_2 \qquad (XI)$$

mit einer Verbindung der allgemeinen Formel (XII)

$$R_{27}NHC(=X)L \qquad (XII)$$

worin eine der Gruppen $R_{26}$ und $R_{27}$ die Gruppe

$$R_1R_2NAlkQO(CH_2)_nCH(OR_5)(CH_2)_m—$$

darstellt und die andere die Gruppe $R_8$ darstellt, und L eine Austrittsgruppe ist, umsetzt,

(d) zur Herstellung von Verbindung der Formel (I), worin $R_5$ für Wasserstoff steht und A für N steht und B für $CR_7$ steht, oder A für $CR_7$ steht und B für N steht, worin $R_7$ die Gruppe $(CH_2)_qR_6$ ist, wobie $R_6$ für $NR_{10}COR_{13}$, $NR_{10}SO_2R_{12}$, $CH_2NHC(=X)NHR_9$ oder $N=CR_{15}R_{16}$ steht, ein Aminoalkyltraizol der Formel (XIV)

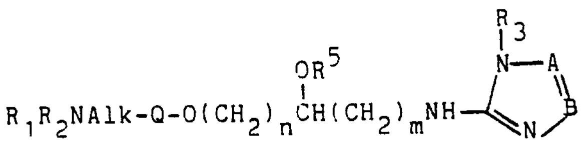
(XIV)

worin $R_1$, $R_2$ und $R_3$ wie in Anspruch 1 definiert sind, oder für Gruppen stehen, die in solche Gruppen leicht umwandelbar sind, A für N steht und B für $CR_{28}$ steht, oder A für $CR_{28}$ steht, und B für N steht, worin $R_{28}$ die Gruppe $(CH_2)_qNHR_{10}$, die Gruppe $(CH_2)_{q+1}NH_2$ oder die Gruppe $(CH_2)_qNH_2$ ist, mit einer Verbindung behandelt, die dazu imstande ist, das Wasserstoffatom in der Gruppe $NHR_{10}$ durch die Gruppe $COR_{13}$ oder $SO_2R_{12}$, oder ein Wasserstoffatom in der Gruppe $NH_2$ der Gruppe $(CH_2)_{q+1}NH_2$ durch die Gruppe $C(=X)NHR_9$, oder beide Wasserstoffatome in der Gruppe $NH_2$ der Gruppe $(CH_2)_qNH_2$ durch die Gruppe $=CR_{15}R_{16}$ zu ersetzen,

(e) zur Herstellung von Verbindungen der Formel (I), worin $R_7$ für eine Acyloxyalkylgruppe steht und/oder $R_5$ für Acyl steht, die entsprechende Verbindung der Formel (I), worin $R_7$ für eine Hydroxyalkylgruppe steht und/oder $R_5$ für Wasserstoff steht, entweder mit einer geeigneten Säure oder einem aktivierten Derivat davon behandelt,

(f) zur Herstellung von Verbindungen der Formel (I), worin A für $CR_7$ steht und B für N steht, $R_3$ und $R_7$ miteinander $—(CH_2)_4—$ darstellen und $R_5$ für Wasserstoff steht, die entsprechende Verbindung, bei der $R_3$ und $R_7$ miteinander $-(CH=CH)_2-$ darstellen, reduziert,

(g) zur Herstellung von Verbindungen der Formel (I), worin Z für

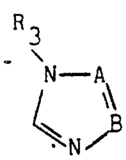

steht, Alk für $CH_2$ steht und $R_5$ für Wasserstoff steht, einen Aldehyd der Formel (XV)

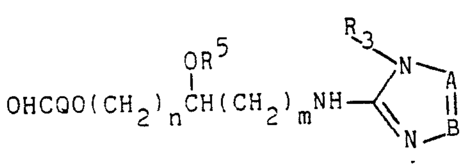
(XV)

mit einem Amin $R_1R_2NH$ behandelt und anschließend reduziert,

(h) zur Herstellung von Verbindungen der Formel (I) worin $R_5$ für Wasserstoff steht und $R_6$ für Tetrahydropyranyloxy steht, das entsprechende Hydroxyalkyltriazol der Formel (i) mit Dihydropyran umsetzt,

(i) zur Herstellung einer Verbindung der Formel (I) in Form eines physiologisch annehmbaren Salzes, die Verbindung der Formel (I) in Form der freien Base in ein physiologisch annehmbares Salz umwandelt.

6. Arzneimittel, dadurch gekennzeichnet, daß es eine Verbindung nach einem der Ansprüche 1 bis 4 zusammen mit mindestens einem inerten pharmazeutische annehmbaren Träger oder Verdünnungsmittel enthält.

7. Verbindungen der allgemeinen Formel (VII)

$$R_1R_2NAlkQO(CH_2)_nCH(OR^5)(CH_2)_mNH_2 \quad (VII)$$

worin $R_1$, $R_2$, Alk, Q, $R_5$, n und m wie in Anspruch 1 definiert sind, wobei die folgende Verbindung ausgenommen ist:

3-(3-Amino-2-hydroxypropoxy)-N,N-dimethylbenylamin.

8. Verbindungen nach Anspruch 7, dadurch gekennzeichnet, daß $R_1$, $R_2$, Alk, Q, $R_5$, n und m wie in Anspruch 2 definiert sind.

9. Verbindungen nach Anspruch 7, dadurch gekennzeichnet, daß $R_1$, $R_2$, Alk, Q, $R_5$, n und m wie in Anspruch 3 definiert sind.

## Revendications

1. Composés de formule générale (I)

$$R_1R_2NAlkQO(CH_2)_nCH(OR_5)(CH_2)_mNH{-}Z \quad (I)$$

et leurs sels et hydrates physiologiquement acceptables dans laquelle:

$R_1$ représente un atome d'hydrogène, un radical alkyl en $C_{1-14}$, cycloalkyle en $C_{3-8}$, alcényle en $C_{3-6}$, alkynyle en $C_{3-6}$, arylalkyle en $C_{1-6}$, trifluoroalkyle en $C_{1-6}$, hétéroalkyle ou un radical alkyle en $C_{1-6}$ substitué par un cycloalkyle en $C_{3-8}$, hydroxy, alcoxy en $C_{1-6}$, amino, alkylamino en $C_{1-6}$ ou dialkylamino en $C_{1-6}$; et

$R_2$ représente un atome d'hydrogène ou un radical alkyle en $C_{1-4}$;

ou bien $R_1$ et $R_2$ ensemble avec l'atome d'azote auquel ils sont fixés, forment un noyau de 5 à 10 éléments qui peut être saturé ou peut contenir au moins une double liaison, peut être non substitué ou substitué par un ou plusieurs radicaux alkyle en $C_{1-3}$ ou un groupe hydroxy et/ou peut contenir un autre hétéroatome choisi parmi l'oxygène ou le soufre;

Alk représente une chaîne alkylène droite ou ramifiée de 1 à 3 atomes de carbone;

Q représente un noyau benzénique dont l'incorporation dans le restant de la molécule se fait par des liaisons dans les positions 1 et 3 ou 1 et 4

$R_5$ représente l'hydrogène ou un radical acyle;

*n* et *m*, qui peuvent être identiques ou différents sont chacun 1 ou 2; Z représente l'un des groupes:

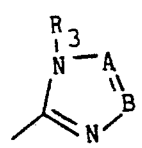 ou 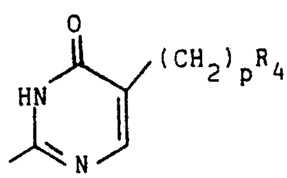 ou 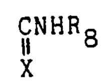

dans lesquels X représente NCN, $NSO_2$Méthyle, $NSO_2$Phényle ou $CHNO_2$;

$R_8$ est un radical alkyle en $C_{1-6}$;

A représente N et B représente $CR_7$ ou

A représente $CR_7$ et B représente N; ou

A et B représentent tous deux N;

$R_3$ représente un atome d'hydrogène, un radical alkyle en $C_{1-6}$, alcényle en $C_{3-6}$, arylalkyle en $C_{1-6}$, hydroxyalkyle en $C_{2-6}$, alcoxy($C_{1-6}$)alkyle en $C_{2-6}$ ou alcanoyloxy($C_{1-4}$)alkyle en $C_{2-6}$;

$R_7$ représente un atome d'hydrogène, un radical alkyle en $C_{1-6}$, alcényle en $C_{3-6}$, arylalkyle en $C_{1-6}$, acyloxyalkyle en $C_{1-6}$, alkylthio($C_{1-6}$)alkyle en $C_{1-6}$, arylthioalkyle en $C_{1-6}$, aryloxyalkyle en $C_{1-6}$, arylalkyloxy($C_{1-6}$)alkyle en $C_{1-6}$ ou le groupe $(CH_2)_qR_6$ dans lequel *q* est 0, 1, 2, 3, 4, 5 ou 6 et la chaîne alkylène $(CH_2)_q$ peut être droite ou ramifiée, et

$R_6$ est un radical hydroxy, alcoxy en $C_{1-6}$, nitro, hétéroaryle, tétrahydropyranyloxy ou $CH_2NHC(=X)NHR_9$ dans lequel X est tel que défini plus haut et $R_9$ est un radical alkyle en $C_{1-6}$;

ou $R_6$ est le groupe $NR_{10}R_{11}$ dans lequel $R_{10}$ est un atome d'hydrogène ou un radical alkyle en $C_{1-6}$ et $R_{11}$ est un atome d'hydrogène, un radical alkyle en $C_{1-6}$, alcényle en $C_{3-6}$, aryle, arylalkyle en $C_{1-6}$ ou hétéroalkyle, ou bien $R_{11}$ est le groupe $SO_2R_{12}$ dans lequel $R_{12}$ est un radical alkyle ou aryle en $C_{1-6}$; ou bien

$R_{11}$ est le groupe $COR_{13}$ dans lequel $R_{13}$ est un atome d'hydrogène, un radical alkyle en $C_{1-6}$, aryle, arylalkyle en $C_{1-6}$, alcoxy en $C_{1-6}$, halométhyle, hétéroaryle, hétéroaralkyle ou le groupe $NHR_{14}$ dans lequel $R_{14}$ est un atome d'hydrogène, un radical alkyle en $C_{1-6}$, cycloalkyle en $C_{3-8}$, aryle ou arylalkyle en $C_{1-6}$; ou $R_{10}$ et $R_{11}$ forment ensemble le groupement $=CR_{15}R_{16}$ dans lequel $R_{15}$ est un radical aryle ou hétéroaryle et $R_{16}$ est un atome d'hydrogène ou un radical alkyle en $C_{1-6}$; ou

$R_6$ est le groupe $SO_2R_{17}$ dans lequel $R_{17}$ est un radical hydroxy, alkyle en $C_{1-6}$, aryle ou le groupe $NR_{18}R_{19}$ dans lequel $R_{18}$ et $R_{19}$, qui peuvent être identiques ou différents, représentent chacun un atome d'hydrogène ou un radical alkyle en $C_{1-6}$; ou bien $R_6$ est le groupe $COR_{20}$ dans lequel $R_{20}$ est un atome d'hydrogène, un radical hydroxy, alcoxy en $C_{1-6}$, aryloxy, arylalkyloxy en $C_{1-6}$, alkyle en $C_{1-6}$, arylalkyle en $C_{1-6}$ ou le groupement $NR_{21}R_{22}$ dans lequel $R_{21}$ est un atome d'hydrogène ou un radical alkyle en $C_{1-6}$ facultativement substitué par un groupe alcoxy en $C_{1-6}$ et $R_{22}$ est un atome d'hydrogène, un radical alkyle en $C_{1-6}$ (facultativement substitué par hydroxy ou un alcoxy en $C_{1-6}$), alcényle en $C_{3-6}$, aryle, arylalkyle en $C_{1-6}$ ou cycloalkyle en $C_{3-8}$, ou le groupe $NR_{21}R_{22}$ forme un noyau de cinq à huit éléments pouvant contenir un autre hétéroatome, par exemple d'oxygène, ou une double liaison et/ou peut être substitué par un radical hydroxy ou un ou deux radicaux alkyle en $C_{1-3}$; ou bien $R_6$ représente $CR_{23}=NR_{24}$ dans lequel $R_{23}$ est un atome d'hydrogène, un radical alkyle en $C_{1-6}$, aryle ou arylalkyle en $C_{1-6}$ et $R_{24}$ est un radical hydroxy, alcoxy en $C_{1-6}$, arylalkyloxy en $C_{1-6}$ ou le groupement $-NHC(=Y)NH_2$ dans lequel Y est un atome d'oxygène ou de soufre,

ou bien quand A représente $CR_7$ et B représente N, alors les groupes $R_3$ et $R_4$ représentent ensemble $-(CH=CH)_2-$ ou $-(CH_2)_4$;

à la condition que (1) quand le groupe $R_6$ contient un atome de carbon par lequel il est lié du groupe alkylène $(CH_2)_q$ alors le nombre total d'atomes de carbone dans le chaîne résultante ne soit pas supérieur à 6 (c'est-à-dire que *q* n'est pas supérieur à 5);

$R_4$ représente le radical phényle, phénoxy ou pyridinyle que peut être facultativement substitué par un ou plusieurs radicaux halogène, alkyle en $C_{1-6}$, alcoxy$(C_{1-6})$alkyle en $C_{1-6}$ ou alcoxy en $C_{1-6}$ ou par un groupe méthylènedioxy;

*p* représente un nombre entier qui est 1, 2 ou 3; aux autres conditions suivantes:

(2) le terme aryle dans les définitions de $R_1$, $R_3$, $R_6$ et $R_7$ désigne le phényle ou le phényle substitué par un ou plusieurs groupes alkyle en $C_{1-3}$ ou alcoxy en $C_{1-6}$ ou des atomes d'halogène;

(3) le terme hétéroaryle désignant un groupe ou un partie d'un groupe dans les définitions de $R_1$ et $R_6$ indique un moyau monocyclique à 5 ou 6 éléments contenant de 1 à 3 hétéro-atomes choisis parmi l'oxygène, l'azote et le soufre, pouvant être non substitué ou substitué par un radical alkyle en $C_{1-3}$, alcoxy en $C_{1-3}$, hydroxyalkyle en $C_{1-6}$ aminoalkyle en $C_{1-6}$, alkyl$(C_{1-6})$aminoalkyle en $C_{1-6}$, dialkyl$(C_{1-6})$aminoalkyle en $C_{1-6}$ ou halogène;

(4) la portion alkyle d'un groupe hétéroalkyle dans les définitions de $R_1$ et $R_6$ est une chaîne alkyle droite ou ramifiée en $C_{1-4}$ et le noyau hétéroarylique est lié à la portion alkyle par un atome carbone;

(5) le terme acyle indiquant un groupe ou une partie d'un groupe dans les définitions de $R_5$ et $R_6$ désigne un groupe aryle- ou aroyl-alcanoyle en $C_{2-7}$ (dans lequel le terme aryle est tel que défini dans la condition (2) ou un groupe alcanoyle en $C_{1-6}$;

(6) les composés suivant sont exclus:

(i) N-[3-[3-pipéridinylméthyl)phénoxy]-2-hydroxypropyl]-N'-methyl-N''-cyanoguanidine;

(ii) N-[3-[3-(diméthylaminométhyl)phénoxy]-2-acétoxypropyl]-N'-méthyl-N''-cyanoguanidine;

(iii) 1-[N-3-[3-(diméthylaminométhyl)phénoxy]-2-hydroxypropylamino]-1-N-méthylamino-2-nitroéthène;

(iv) 1-[N-3-[3-(1'-cyclopropylméthylaminométhyl)phénoxy]-2-hydroxy-propylamino]-1-N-méthylamino-2-nitroéthène;

(v) 2-N-[3-[3-(diméthylaminométhyl)phénoxy]-2-hydroxypropyl]amino-5-[(6-méthyl-3-pyridyl)méthyl]-pyrimidinin-4(3H)-one;

(vi) N-3-[3-diméthylaminométhyl)phénoxy-2-hydropropyl]-N'-méthyl-N''-cyanoguanidine.

2. Composé selon la revendication 1, dans lesquels

$R_1$ représente un radical alkyle en $C_{1-8}$, alkyle en $C_{1-4}$ substitué par un groupe trifluorométhyle, alkyle en $C_{2-4}$ substitué par hydroxy ou un groupe dialkyl $(C_{1-3})$amino, cycloalkyle en $C_{5-7}$, alcényle en $C_{3-5}$, phénylalkyle en $C_{1-3}$ ou hétéroarylalkyle en $C_{1-3}$ dans lequel le noyau hétéroaryle contient un hétéroatome;

$R_2$ représente un atome d'hydrogène ou le radical méthyle; ou

$R_1R_2N$ représente un noyau de 5 à 7 éléments contenant facultativement une double liaison, un atome d'oxygène ou un substituant alkyle en $C_{1-6}$;

Alkyl représente le méthylène;

$R_3$ représente un atome d'hydrogène, un radical alkyle en $C_{1-6}$ ou hydroxyalkyle en $C_{2-4}$ ou bien quand A est le groupe $CR_7$, alors $R_3$ et $R_7$ ensemble représentent un groupe $-(CH=CH)_2-$ ou $-(CH_2)_4-$;

Q représente un noyau benzénique incorporé dans le restant de la molécule par des liaisons en positions 1 et 3;

$R_5$ représente un atome d'hydrogène ou un radical alcanoyle en $C_{1-6}$;

*n* et *m* représentent tous les deux 1 ou bien *n* ou *m* représente 2;

$R_7$ est un radical alcoxy $(C_{1-6})$alkyle en $C_{1-6}$; alkylthio$(C_{1-6})$alkyle en $C_{1-6}$, alcanoyloxy $(C_{1-6})$alkyle en $C_{1-6}$, benzène ou $-CH=NOH$;

ou $R_7$ représente le groupe $(CH_2)_qR_6$ dans lequel *q* est 0, 1, 2 ou 3 et $R_6$ représente un radical hydroxyle, $CH_2NHSO_2R_{12}$ (dans lequel $R_{12}$ est alkyle en $C_{1-6}$); $CH_2NHC(=X)NHCH_3$ dans lequel X = NCN ou $CHNO_2$) $SO_2R_{17}$ (dans lequel $R_{17}$ est un radical alkyle en $C_{1-6}$), ou bien $R_6$ représente $COR_{20}$ dans lequel $R_{20}$ est l'hydroxyle ou $NR_{21}R_{22}$ et $R_{21}$ et $R_{22}$ représentent indépendamment un atome d'hydrogène ou un radical alkyle en $C_{1-3}$ ou bien $R_{21}$ et $R_{22}$ ensemble avec l'atome d'azote auxquels ils sont fixés représentent un noyau pyrrolidino, ou

$R_6$ représente le groupe $NR_{10}R_{11}$ dans lequel $R_{10}$ est un atome d'hydrogène et $R_{11}$ est un atome d'hydrogène ou le groupe $COR_{13}$ dans lequel $R_{13}$ représente un atome d'hydrogène, un radical alkyle en $C_{1-6}$, phényle, benzyle alcoxy en $C_{1-6}$, NH-phényle ou bien $R_6$ et $R_{11}$ ensemble représentent le groupe $=CHR_{15}$ dans lequel $R_{15}$ est le phényle ou le pyridyle;

$R_4$ représente un radical pyridinyle facultativement substitué par un alkyle en $C_{1-6}$, ou un groupe phényle facultativement substitué par un alcoxy en $C_{1-6}$;

*p* représente 1;

$R_8$ représente un alkyle en $C_{1-6}$;

X représente $CHNO_2$.

3. Composés selon la revendication 1, de formule générale (II)

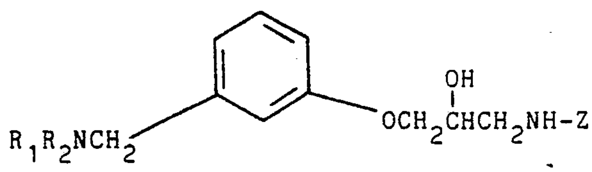

(II)

dans laquelle $R_1$, $R_2N$ est un groupe diméthylamino, pyrrolidino, pipéridino ou hexaméthylèneimino; et Z représente:

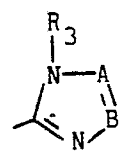 ou 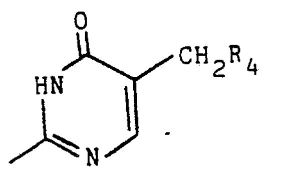 ou 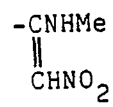

dans laquelle, quand B représente $CR_7$ alors $R_3$ est un atome d'hydrogène ou un radical méthyle et $R_7$ est un groupe hydroxyméthyle, amino, alcanoyl($C_{1-6}$)oxyalkyle en $C_{1-6}$, N = CHPh, $(CH_2)_3NHC(=CHNO_2)NHMe$, aminopropyle, méthylsulfonylméthyle ou acétylamino; ou quand A représente $CR_7$ alors $R_3$ est un radical méthyle et $R_7$ un radical amino ou bien $R_3$ et $R_7$ ensemble forment le groupe $\leftarrow CH=CH \rightarrow_2$ ou $(CH_2)_4$; ou A et B représentent tous deux N, alors $R_3$ est un radical méthyle; et $R_4$ est un radical 3-pyridinyle, 6-méthyl-3-pyridinyle ou 4-méthoxyphényle.

4. 1-[3-Amino-1-méthyl-1H-1,2,4-triazol-5-yl)amino]-3-[3-(1-pipéridinylméthyl)phénoxy]-2-propanol;

5-[[2-hydroxy-3-[3-(1-pipéridinylméthyl)phénoxy]propyl]amino]-1-méthyl-1*H*-1,2,4-triazole-3-méthanol;

1-[(1-méthyl-1H-tétrazol-5-yl)amino]-3-[3-(1-pipéridinylméthyl)-phénoxy]-2-propanol;

2-[[2-hydroxy-3-[3-(1-pipéridinylméthyl]phénoxy]propyl]amino]-5-[(6-méthyl-3-pyridinyl)méthyl]-4-(3*H*)-pyrimidinone;

2-[[2-hydroxy-3-[3-(1-pipéridinylméthyl)phénoxy]propyl]amino]-5-(3-pyridinylméthyl)-4-(3H)-pyrimidinone;

2-[[2-hydroxy-3-[3-(1-pipéridinylméthyl)phénoxy]propyl]amino]-5-[(4-méthoxyphényl)methyl]-4-(3H)-pyrimidinone; et leurs sels physiologiquement acceptables.

5. Procédé de préparation de composés selon la revendication 1, qui consiste:

(a) pour la préparation de composés de formule (I) dans laquelle $R_5$ représente l'hydrogène et Z représente le groupe:

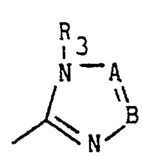

dans lequel ou bien A représente $CR_7$ et B représente N ou bien A représente N et B représente $CR_7$, et $R_7$ est autre que acyloxyalkyle, alcoxy, nitro, $(CH_2)_qN=CR_{15}R_{16}$, $SO_2R_{17}$, $COR_{20}$ (dans laquelle $R_{20}$ est l'hydrogène, l'aryle ou aralkyle) ou $CR_{23}=NR_{24}$, ou bien quand A représente $CR_7$ et B représente N, alors $R_3$ et $R_7$ ne peuvent pas représenter le groupe $\leftarrow CH=CH \rightarrow_2$ ou $-(CH_2)_4-$, à cycliser un composé de formule (III):

$$R_1R_2NAlkQO(CH_2)_nCH(OR_5)(CH_2)_mNHC(=V)—N(R_{25})—NHY' \qquad (III)$$

dans laquelle $R_{25}$ est tel que défini dans la revendication 1 pour $R_3$, V' représente

$$NCR_7'(=V)$$

ou NCN et Y' représentent un atome d'hydrogène quand V est l'oxygène ou le soufre et $R_7'$ est un groupe tel que défini dans la revendication 1 pour $R_7$ ou un groupe convertible en celui-ci dans des conditions de réaction de cyclisation ou bien $R_7'$ représente un halogène ou alcoxy; ou V' est $NR_3$, $R_{25}$ est un atome d'hydrogène et Y' est

$$CR_7(=Y'')$$

dans lequel Y'' est une atome de soufre, d'oxygène ou NH;

(b) pour la préparation de composés de formule (I) dans laquelle $R_5$ est un atome d'hydrogène et Z est tel que défini dans la revendication 1 sauf $C(=X)NHR_8$ et sauf que lorsque A ou B représente le groupe $CR_7$, alors $CR_7$ représente nitro ou quand A représente $CR_7$ alors $R_3$ et $R_7$ ensemble représentent le groupe $-(CH=CH)_2-$, à chauffer le diamine (VII):

$$R_1R_2NAlkQO(CH_2)_nCH(OR_5)(CH_2)_mNH_2 \qquad (VII)$$

dans laquelle $R_5$ représente un atome d'hydrogène avec un composé de formule (IX) ou (X):

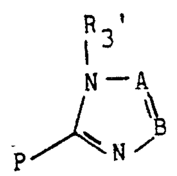

(IX)

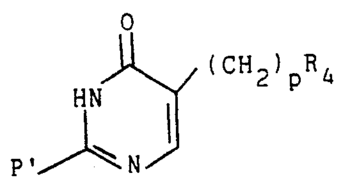

(X)

dans laquelle $R_3'$ est le groupe $R_3$ ou un groupe convertible en celui-ci et P et P' sont des groupes partants;

(c) pour la production de composés de formule (I) dans laquelle Z représente:

$$—C(=X)NHR_8$$

et $R_5$ représente un atome d'hydrogène, à faire réagir une amine de formule (XI):

$$R_{26}NH_2 \qquad (XI)$$

avec un composé de formule générale (XII)

$$R_{27}NHC(=X)L \qquad (XII)$$

dans laquelle l'un des groupes $R_{26}$ ou $R_{27}$ représente le groupe

$$R_1R_2NAlkQO(CH_2)_n\overset{\overset{OR_5}{|}}{C}H(CH_2)_m—$$

et l'autre représente le groupe $R_8$; et L est un groupe partant;

(d) pour la production des composés de formule (I) dans laquelle $R_5$ représente un atome d'hydrogène et A représente N et B représente $CR_7$ ou bien A représente $CR_7$ et B représente N, dans laquelle $R_7$ est le groupe $(CH_2)_qR_6$ dans lequel $R_6$ est $NR_{10}COR_{13}$, $NR_{10}SO_2R_{12}$, $CH_2NHC(=X)NHR_9$ ou $N=CR_{15}R_{16}$, à traiter une aminoalkyltriazole de formule (XIV):

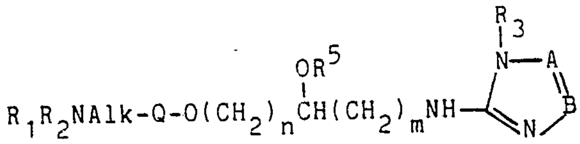

(XIV)

dans laquelle $R_1$, $R_2$ et $R_3$ sont tels que définis dans la revendication 1 ou sont des groupes facilement convertibles en ceux-ci, A représente N et B représente $CR_{28}$ ou A représente $CR_{28}$ et B représente N, dans laquelle $R_{28}$ est le groupe $(CH_2)_qNHR_{10}$, le groupe $(CH_2)_{q+1}NH_2$ ou le groupe $(CH_2)_qNH_2$, avec un composé capable de remplacer l'atome d'hydrogène dans le groupe $NHR_{10}$ par le groupe $COR_{13}$ ou $SO_2R_{12}$ ou un atome d'hydrogène dans le groupe $NH_2$ du groupe $(CH_2)_{q+1}NH_2$ par le groupe $C(=X)NHR_9$ ou les deux atomes d'hydrogène dans le groupe $NH_2$ du groupe $(CH_2)_9NH_2$ par le groupe $=CR_{15}R_{16}$;

e) pour la production de composés de formule (I) dans lesquels $R_7$ est un groupe acyloxyalkyle et/ou $R_5$ est un groupe acyle, à traiter le composé correspondant de formule (I) dans lequel $R_7$ est un groupe hydroxyalkyle et/ou $R_5$ est un atome d'hydrogène avec un acide approprié ou un dérivé activé de celui-ci;

(f) pour la production de composés de formule (I) dans lesquels A est $CR_7$ et B est N, $R_3$ et $R_7$ ensemble représentent $—(CH_2)_4—$ et $R_5$ est un atome d'hydrogène, à réduire le composé correspondant dans lequel $R_3$ et $R_7$ représentent ensemble $\leftarrow CH=CH \rightarrow_2$;

(g) pour la production de composé de formule (I) dans lesquels Z est:

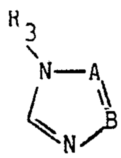

Alk est $CH_2$ et $R_5$ est un atome d'hydrogène, à traiter un aldéhyde de formule (XV):

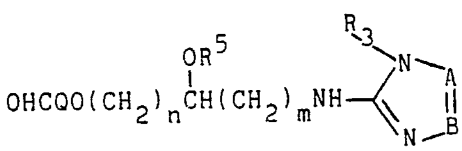

(XV)

avec une amine $R_1R_2NH$, qu'on fait suivre d'une réduction;

(h) pour la production de composés de formule (I) dans lesquels $R_5$ est un atome d'hydrogène et $R_6$ est le tétrahydropyranyloxy, à faire réagir le hydroxyalkyltriazole correspondant de formule (I) avec le dihydropyrane;

(i) pour la production d'un composé de formule (I) sous forme d'un sel physiologiquement acceptable, à convertir le composé de formule (I) sous forme de base libre en un sel physiologiquement acceptable.

6. Composition pharmaceutique que comprend un composé selon l'une quelconque des revendications 1 à 4, ensemble avec au moins un véhicule ou diluant inerte pharmaceutiquement acceptable.

7. Composés de formule générale (VII)

$$R_1R_2NAlkQO(CH_2)_n\overset{\overset{OR^5}{|}}{C}H(CH_2)_mNH_2 \qquad (VII)$$

dans laquelle $R_1$, $R_2$, Alk, Q, $R_5$, *n* et *m* sont tels que définis dans la revendication 1; le composé suivant étant exclu:

3-(3-amino-2-hydroxypropoxy)-N,N-diméthylbenzylamine.

8. Composés selon la revendication 7, dans lesquels $R_1$, $R_2$, Alk, Q, $R_5$, *n* et *m* sont tels que définis dans la revendication 2.

9. Composés selon la revendication 7, dans lesquels $R_1$, $R_2$, Alk, Q, $R_5$, *n* et *m* sont tels que définis dans la revendication 3.